# EUROPEAN PATENT APPLICATION

(11) **EP 4 218 821 A2**
(43) Date of publication of application: **02.08.2023**
(21) Application number: 23153918.0
(22) Date of filing: 30.07.2019
(51) Int. Cl.: A61K 47/26, A61K 39/00, A61P 35/00

(54) **PHARMACEUTICAL FORMULATIONS OF MASKED ANTIBODIES**

(30) Priority: 31.07.2018 US 201862712906 P
(62) Divisional of application: 19759082.1
(71) Applicant: Amgen Inc., Thousand Oaks, CA 91320-1799 (US)
(72) Inventor: TREUHEIT, Michael John, California, 91320-1799 (US); GHATTYVENKATAKRISHNA, Pavan, California, 91320-1799 (US); JAGANNATHAN, Bharadwaj, California, 91320-1799 (US)
(74) Representative: J A Kemp LLP

(57) **Abstract**

The present disclosure is directed to low pH pharmaceutical compositions and/or formulations comprising a masked antigen binding protein.

## Description

### CROSS-REFERENCE TO RELATED APPLICATION

This application claims the benefit of U.S. Provisional Application No. 62/712,906, filed on July 31, 2018, which is hereby incorporated by reference.

### FIELD OF THE INVENTION

The present disclosure is directed to low pH pharmaceutical compositions and/or formulations comprising a masked antigen binding protein.

### CROSS-REFERENCE TO MATERIAL INCORPORATED BY REFERENCE

Incorporated by reference in its entirety is a computer-readable nucleotide/amino acid sequence listing submitted concurrently herewith and identified as follows: ASCII (text) file named "52811_Seqlisting.txt", Size: 12,007 bytes; Created: July 30, 2018.

### BACKGROUND

Protein-based pharmaceuticals are among the fastest growing therapeutic agents in (pre)clinical development and as commercial products. In comparison with small chemical drugs, protein pharmaceuticals have high specificity and activity at relatively low concentrations, and typically provide for therapy of high impact diseases such as various cancers, auto-immune diseases, and metabolic disorders (Roberts, Trends Biotechnol. 2014 Jul;32(7):372-80, Wang, Int J Pharm. 1999 Aug 20;185(2):129-88).

Protein-based pharmaceuticals, such as recombinant proteins and antibodies, can now be obtained in high purity when manufactured due to advances in commercial scale purification processes. However, recombinant proteins, including antibodies, are only marginally stable and are highly susceptible to both chemical and physical degradation. Chemical degradation refers to modifications involving covalent bonds, such as deamidation, oxidation, cleavage or formation of new disulfide bridges, hydrolysis, isomerization, or deglycosylation. Physical degradation includes protein unfolding, undesirable adsorption to surfaces, and aggregation. Dealing with these physical and chemical instabilities is one of the most challenging tasks in the development of protein pharmaceuticals (Chi et al., Pharm Res, Vol. 20, No. 9, Sept 2003, pp. 1325-1336, Roberts, Trends Biotechnol. 2014 Jul;32(7):372-80).

Protein aggregation represents a major event of physical instability of proteins and antibodies, and generally occurs due to the inherent tendency to minimize the thermodynamically unfavorable interaction between solvent and hydrophobic protein residues. It is particularly problematic because it is encountered routinely during production, refolding, purification, sterilization, shipping, and storage processes. Aggregation can occur even under solution conditions where the protein or antibody native state is highly thermodynamically favored (e.g., neutral pH and 37°C) and in the absence of stresses (Chi et al., Pharm Res, Vol. 20, No. 9, Sept 2003, pp. 1325-1336, Roberts, Trends Biotechnol. 2014 Jul;32(7):372-80, Wang, Int J Pharm. 1999 Aug 20;185(2):129-88, Mahler J Pharm Sci. 2009 Sep;98(9):2909-34).

Antibody aggregation is problematic because it can impair biological activity. Moreover, aggregation of antibodies leads to undesirable aesthetics of the drug product and decreases product yield due to elaborate purification steps required to remove the aggregates from the end product. More recently, there has also been growing concern that aggregated antibodies (even humanized or fully human antibodies) can significantly increase the risk that a patient will develop an immune response to the active antibody/protein monomer, resulting in the formation of neutralizing antibodies and drug resistance, or other adverse side effects (Mahler J Pharm Sci. 2009 Sep;98(9):2909-34).

In general, several efforts have been reported to minimize protein aggregation by various mechanisms. Proteins, including antibodies, can also be stabilized and thus protected from aggregate formation and other chemical changes by modifying their primary structure, thereby increasing interior hydrophobicity and reducing outer hydrophobicity. However, genetic engineering of proteins or antibodies may result in impaired functionality and/or increased immunogenicity. Another approach focuses on the dissociation of aggregates (referred to as "disaggregation") to recover functional, native monomers by using various mechanisms such as temperature, pressure, pH, and salts. Currently, protein and antibody aggregates are removed as impurities mainly in the polishing steps of downstream processing. However, in cases of high levels of high-molecular weight (HMW) antibody species, removing significant amount of HMW not only results in substantial yield loss, but also makes the design of a robust downstream process challenging (Chi et al., Pharm Res, Vol. 20, No. 9, Sept 2003, pp. 1325-1336).

Preserving protein stability and activity in biological and biotechnological applications poses serious challenges. There is a need in the art for optimized pharmaceutical compositions that provide for enhanced stabilization of antibodies or therapeutic proteins and reduce aggregation and denaturation or degradation during formulation, filling, shipping, storage and administration, thereby preventing loss-of-function and adverse immunogenic reactions.

### SUMMARY

The present disclosure provides pharmaceutical compositions and/or formulations useful to minimize or reduce aggregation and/or high-molecular weight species of masked antigen binding proteins. In some embodiments, the present disclosure provides a pharmaceutical composition or formulation comprising: (A) a masked antigen binding protein (including, e.g., a Probody or a ProTIA prodrug) comprising: (i) an antibody or antigen binding fragment thereof (AB1) that binds to an antigen; and (ii) a masking domain (MD1) coupled to AB1, wherein the masking domain comprises: (a) a masking peptide or masking polypeptide (MP1) that inhibits or reduces the binding of AB1 to the antigen; and (b) a protein recognition site (PR1), wherein binding to or cleavage of the PR1 by a protein or a protease increases AB1 binding to the antigen; (B) at least one buffer agent; and (C) at least one polyol, wherein the pH of the pharmaceutical composition or formulation is below the isoelectric point (pI) of the AB1 and MD1 regions of the masked antigen binding protein.

In some other embodiments, the present disclosure also provides a pharmaceutical composition or formulation comprising: (A) a masked antigen binding protein (including, e.g., a Probody or a ProTIA prodrug) comprising: (i) a first antibody or antigen binding fragment thereof (AB1) that binds to an antigen; and (ii) a first masking domain (MD1) coupled to AB1, wherein the masking domain comprises: (a) a masking peptide or masking polypeptide (MP1) that inhibits or reduces the binding of AB1 to the antigen; and (b) a protein recognition site (PR1), wherein binding to or cleavage of the PR1 by a protein or a protease increases AB1 binding to the antigen; (iii) a second antibody or antigen binding fragment thereof (AB2) that binds to a second antigen; and (vi) a second masking domain (MD2) coupled to AB2, wherein the second masking domain comprises: (a) a masking peptide or masking polypeptide (MP2) that inhibits or reduces the binding of AB2 to the antigen; and (b) a second protein recognition site (PR2), wherein binding to or cleavage of the PR2 by a protein or a protease increases AB2 binding to the second antigen, (B) at least one buffer agent; and (C) at least one polyol, wherein the pH of the pharmaceutical composition or formulation is below the isoelectric points (pI) of the AB1, AB2, MD1 and MD2 regions of the masked antigen binding protein.

In some aspects, the AB1 and/or AB2 regions of the masked antigen binding protein (including, e.g., a Probody or a ProTIA prodrug) is an scFv or a Fab, and/or binds an antigen expressed on the surface of a cell (including, e.g., CD3).

The pharmaceutical compositions or formulations of the disclosure comprises at least one buffer agent. In some embodiments, the buffer agent is selected from the group consisting of acetate, glutamate, citrate, succinate, tartrate, fumarate, maleate, histidine, phosphate, and 2-(N-morpholino)ethanesulfonate or a combination thereof. In some embodiments, the at least one buffer agent is present in the composition or formulation at a concentration ranging from about 5 mM to about 200 mM (e.g., 5 mM to 200 mM) or about 10 mM to about 50 mM (e.g., 10 mM to 50 mM).

The pharmaceutical compositions or formulations of the disclosure also comprises at least one polyol. In some embodiments, the polyol is a saccharide. In some embodiments, the saccharide is selected from the group consisting of a monosaccharide, a disaccharide, a cyclic polysaccharide, a sugar alcohol, a linear branched dextran, and a linear non-branched dextran, or combinations thereof. In some embodiments, the polyol is a sugar alcohol (e.g., sorbitol). In some embodiments, the polyol is sucrose, trehalose, mannitol, sorbitol, xylitol, erythritol, isomalt, glycerol, a cyclodextrin or captisol. In some embodiments, the at least one polyol is present in the composition or formulation at a concentration ranging from about 1 to about 20% (w/V) (or about 9 to about 12% (w/V)).

In some instances, the pharmaceutical compositions or formulations of the disclosure may optionally further comprise at least one surfactant. In some embodiments, the surfactant is selected from the group consisting of polysorbate 20, polysorbate 40, polysorbate 60, polysorbate 80, poloxamer 188, pluronic F68, triton X-100, polyoxyethylen3, and PEG 3350, PEG 4000, PEG 8000 or a combination thereof. In some embodiments, the at least one surfactant is present in the composition at a concentration ranging from 0.004 to about 0.5% (w/V) (or about 0.001 to about 0.01% (w/V)).

In some embodiments, the pH of the composition or formulation is greater than 3.5, and/or the pH is about 2 pH units lower than the pI of AB1, AB2, MD1, and/or MD2 region(s) (described further below) of the masked antigen binding protein (including, e.g., a Probody or a ProTIA prodrug). In some embodiments, the pH of the composition or formulation is between 7.0 and 3.6. In some embodiments, the pH of the composition or formulation ranges from about 3.5 to about 5.5 or ranges from 4.0 to 5.0. In some embodiments, the pH of the composition or formulation is about 4.2 or about 3.6. In some embodiments, the pH of the composition or formulation is 4.2 or 3.6.

The pharmaceutical compositions or formulations of the disclosure may optionally further comprise an amino acid excipient. In some embodiments, the amino acid excipient is present at a concentration ranging from about 0.1 to about 20% (mM).

The pharmaceutical composition or formulations of the disclosure, in some embodiments, comprises 10 mM glutamate, 9% (w/V) sucrose and 0.01% (w/V) polysorbate 80, and wherein the pH of the liquid pharmaceutical composition is 4.2 or 3.6. In some embodiments, the masked antigen binding protein is present in the composition at a concentration ranging from about 0.1 mg/mL to about 17 mg/mL or from about 0.1 mg/mL to about 30 mg/mL. In some embodiments, the masked antigen binding protein is present in the composition in an amount ranging from about 50 µg to about 200 mg.

The pharmaceutical compositions of the disclosure can be a lyophilized composition or a liquid composition. In some embodiments, the pharmaceutical composition is a reconstituted lyophilized composition.

In some embodiments, the pharmaceutical composition is a liquid composition that is stable after storage at 2-8°C, 4°C, and/or 25°C for 0, 1, 2 and/or 4 weeks. In some embodiments, the pharmaceutical composition or formulation has reduced antibody aggregation and/or reduced high-molecular weight antibody species as measured by ultra-high performance liquid chromatography (SE-UHPLC) of about 1-fold, about 2-fold, about 3-fold, about 4-fold, about 5-fold, about 6-fold, about 7-fold, about 8-fold, about 9-fold or about 10-fold compared to a reference pharmaceutical composition consisting of 1 mg/ml masked antigen binding protein, 100 mM glycine at pH 4.9.

In another aspect, described herein is a method of treating cancer in a subject in need thereof comprising administering a composition or formulation of the disclosure to the subject.

The terms "comprising," "having," "including," and "containing" are to be construed as open-ended terms (i.e., meaning "including, but not limited to," and permit the presence of one or more features or components) unless otherwise noted. It should be understood that while various embodiments in the specification are presented using "comprising" language, under various circumstances, a related embodiment may also be described using "consisting of" or "consisting essentially of" language. It is to be noted that the term "a" or "an" refers to one or more, for example, "an immunoglobulin molecule," is understood to represent one or more immunoglobulin molecules, unless context dictates otherwise. As such, the terms "a" (or "an"), "one or more," and "at least one" can be used interchangeably herein. Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It should also be understood that when describing a range of values, the characteristic being described could be an individual value found within the range. For example, "a pH from about pH 4 to about pH 6," could be, but is not limited to, pH 4, 4.2, 4.6, 5.1, 5.5, etc. and any value in between such values. Additionally, "a pH from about pH 4 to about pH 6," should not be construed to mean that the pH of a formulation in question varies 2 pH units in the range from pH 4 to pH 6 during storage, but rather a value may be picked in that range for the pH of the solution, and the pH remains buffered at about that pH. In some embodiments, when the term "about" is used, it means the recited number plus or minus 10% of that recited number.

In any of the ranges described herein, the endpoints of the range are included in the range. However, the description also contemplates the same ranges in which the lower and/or the higher endpoint is excluded. Additional features and variations of the invention will be apparent to those skilled in the art from the entirety of this application, including the drawing and detailed description, and all such features are intended as aspects of the invention. Likewise, features of the invention described herein can be re-combined into additional embodiments that also are intended as aspects of the invention, irrespective of whether the combination of features is specifically mentioned above as an aspect or embodiment of the invention. Also, only such limitations which are described herein as critical to the invention should be viewed as such; variations of the invention lacking limitations which have not been described herein as critical are intended as aspects of the invention.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various aspects or aspects of the disclosure, which can be had by reference to the specification as a whole.

All references cited herein are hereby incorporated by reference in their entireties.

### BRIEF DESCRIPTION OF THE DRAWINGS

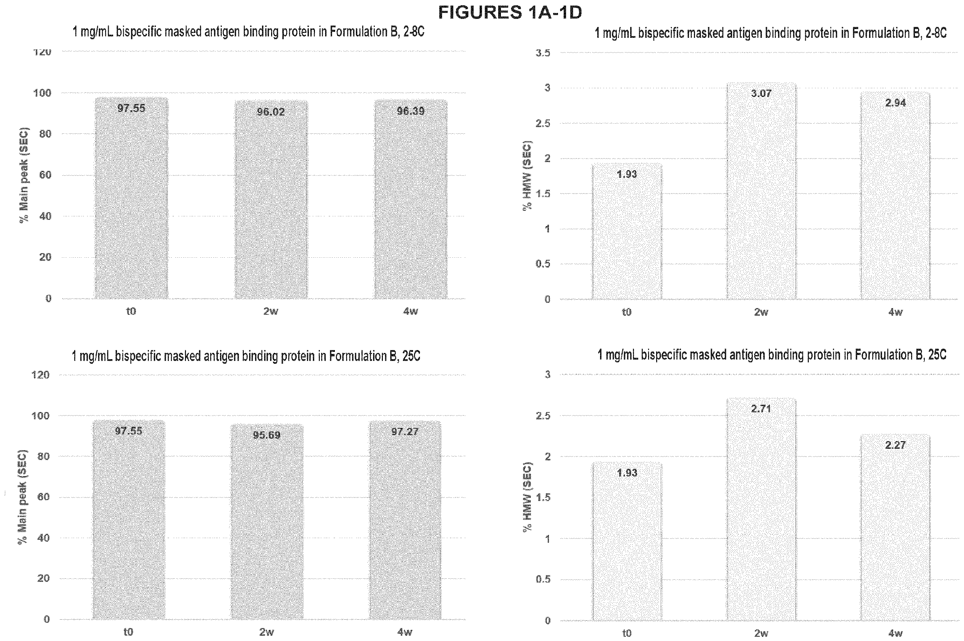

**Figures 1A-1D** are bar graphs providing the results of size exclusion ultra-high-performance liquid chromatography (SE-UHPLC) analysis, showing the % Main Peak (left panels) and % high molecular weight species (right panels) of the bispecific masked antigen binding protein in Formulation B, when stored at either 2-8°C or 25°C at 0, 2 weeks and 4 weeks.

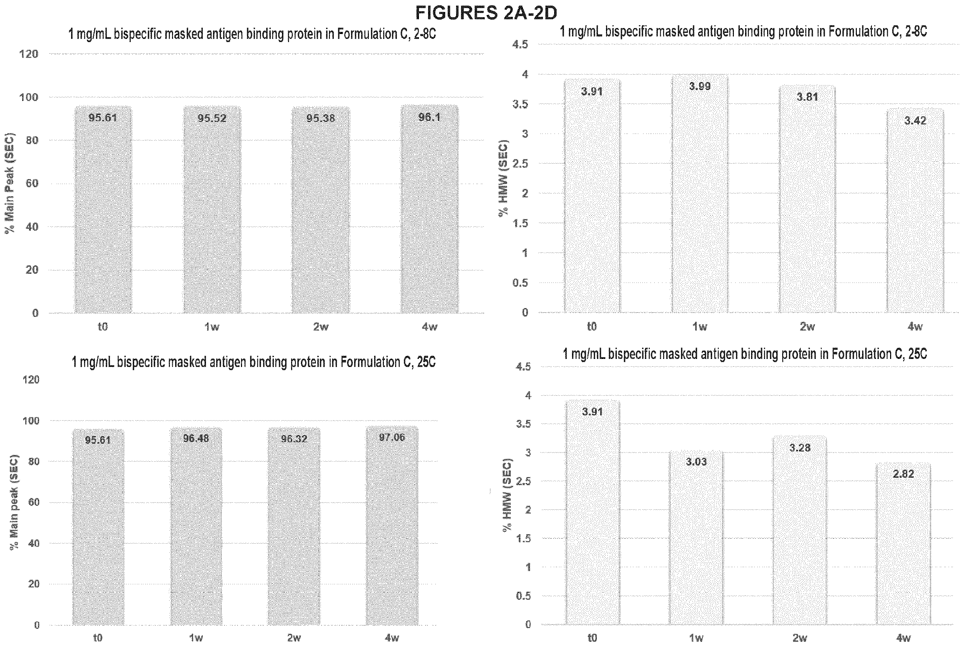

**Figures 2A-2D** are bar graphs providing the results of SE-UHPLC analysis, showing the % Main Peak (left panels) and % high molecular weight species (right panels) of the bispecific masked antigen binding protein in Formulation C, when stored at either 2-8°C or 25°C at 0, 1 week, 2 weeks and 4 weeks.

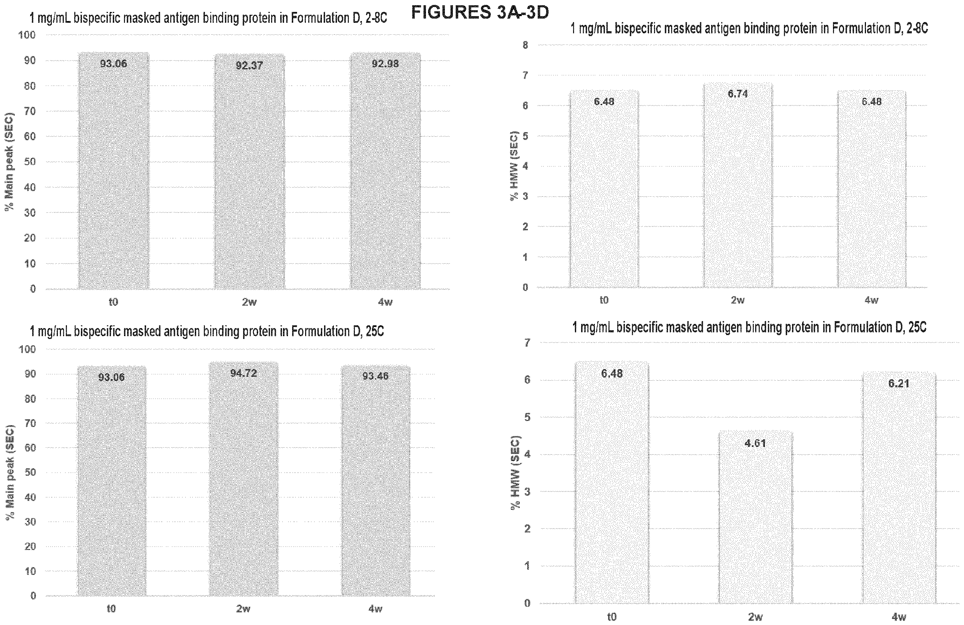

**Figures 3A-3D** are bar graphs providing the results of SE-UHPLC analysis, showing the % Main Peak (left panels) and % high molecular weight species (right panels) of the bispecific masked antigen binding protein in Formulation D, when stored at either 2-8°C or 25°C at 0, 2 weeks and 4 weeks.

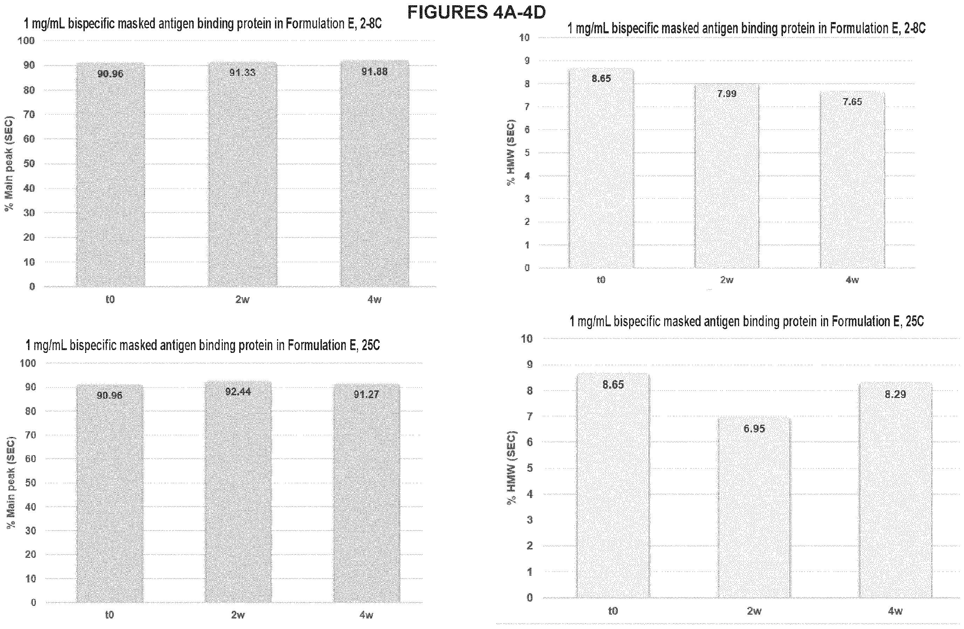

**Figures 4A-4D** are bar graphs providing the results of SE-UHPLC analysis, showing the % Main Peak (left panels) and % high molecular weight species (right panels) of the bispecific masked antigen binding protein in Formulation E, when stored at either 2-8°C or 25°C at 0, 2 weeks and 4 weeks.

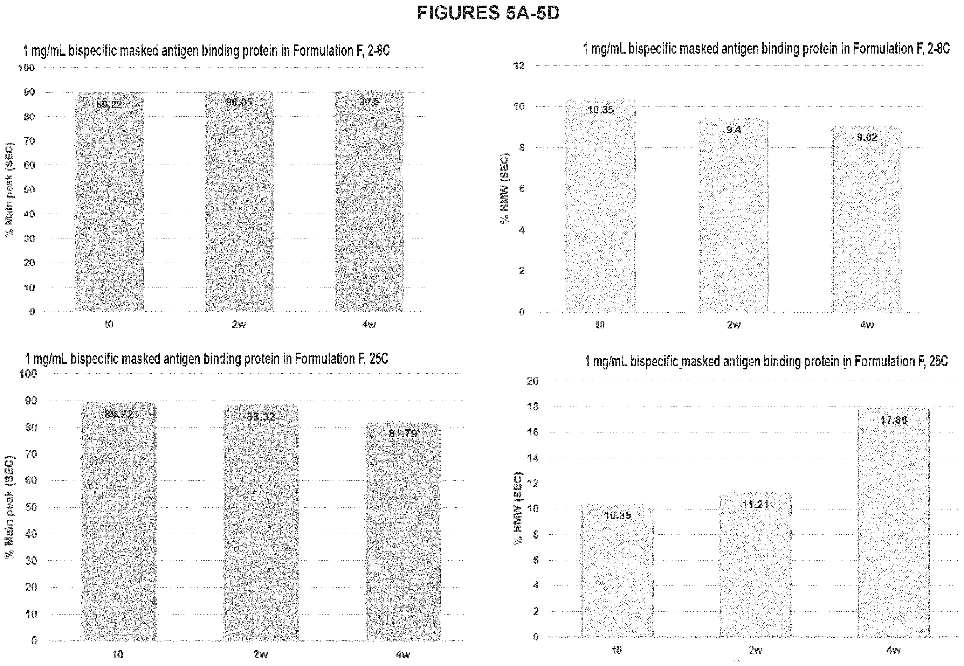

**Figures 5A-5D** are bar graphs providing the results of SE-UHPLC analysis, showing the % Main Peak (left panels) and % high molecular weight species (right panels) of the bispecific masked antigen binding protein in Formulation F, when stored at either 2-8°C or 25°C at 0, 2 weeks and 4 weeks.

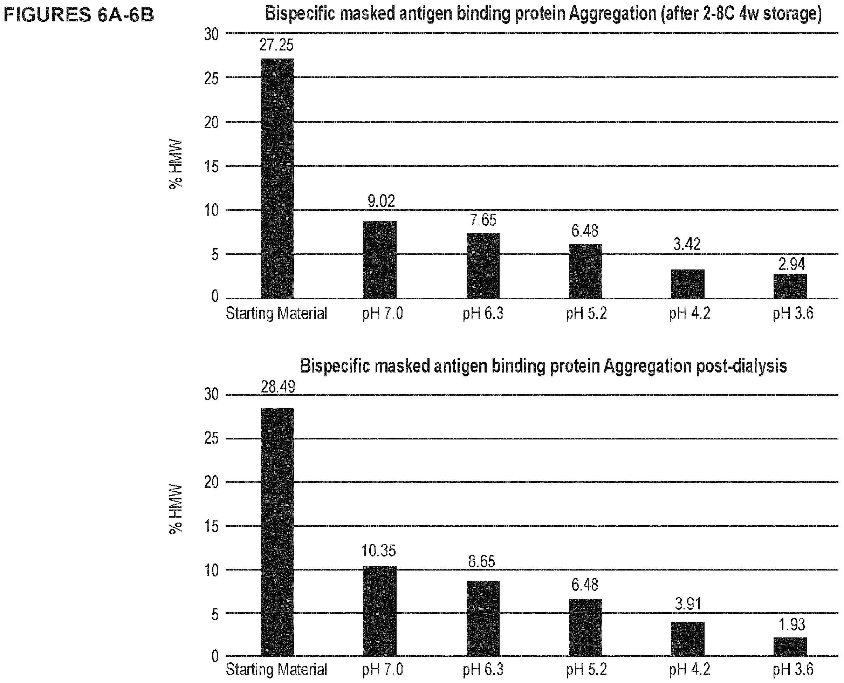

**Figures 6A and 6B** are bar graphs providing the results of SE-UHPLC analysis, showing the % high molecular weight species of the bispecific masked antigen binding protein in Formulation A ("Starting Material"), Formulation F ("pH 7.0"), Formulation E ("pH 6.3"), Formulation D ("pH 5.2"), Formulation C ("pH 4.2") or Formulation B ("pH 3.6"), when stored at either 2-8°C at 0 (bottom panels) and 4 weeks (top panels).

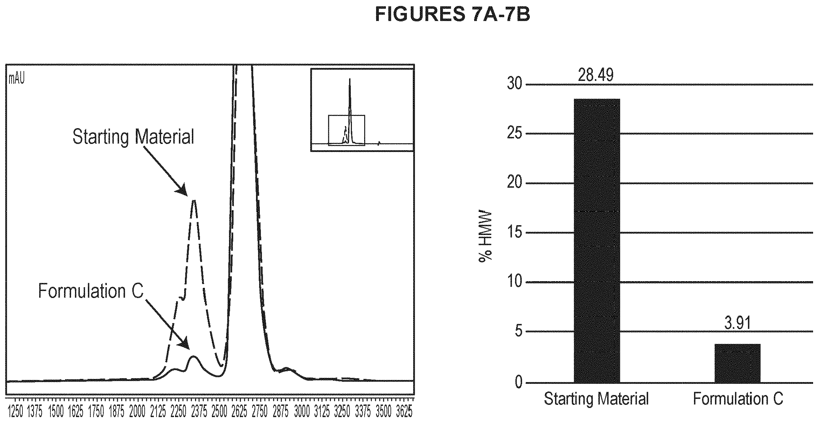

**Figures 7A and 7B** are graphs showing a decrease in aggregation after dialysis into a low pH formulation (Formulation C) compared to starting material (Formulation A).

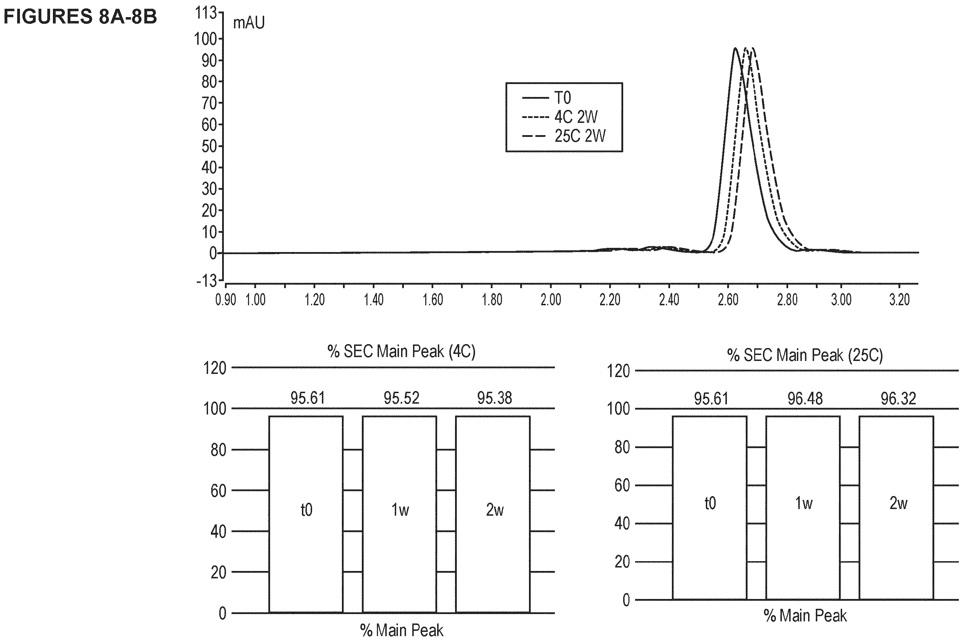

**Figures 8A and B**are graphs showing the stability of the bispecific masked antigen binding protein (1 mg/mL) in a low pH formulation (Formulation C) when stored at either 4°C or 25°C for 0, 1 weeks or two weeks, as determined by size exclusion analysis. In Figure 8A the chromatograms are offset.

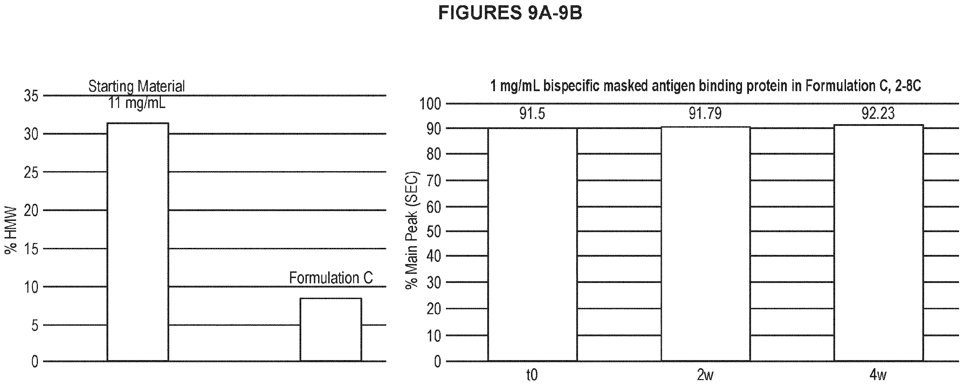

**Figures 9A and B**are bar graphs showing the stability of the bispecific masked antigen binding protein in low pH formulation as high concentrations (e.g., 17 mg/mL), as determined by size-exclusion (SEC) analysis. Formulation C at a protein concentration of 17 mg/mL had significantly lower aggregation / % high molecular weight species compared to Formulation A ("Starting Material") at a protein concentration of 11 mg/mL (left panel). Formulation C at a protein concentration of 17 mg/mL was also stable after 0, two or four weeks of storage at 4°C (right panel).

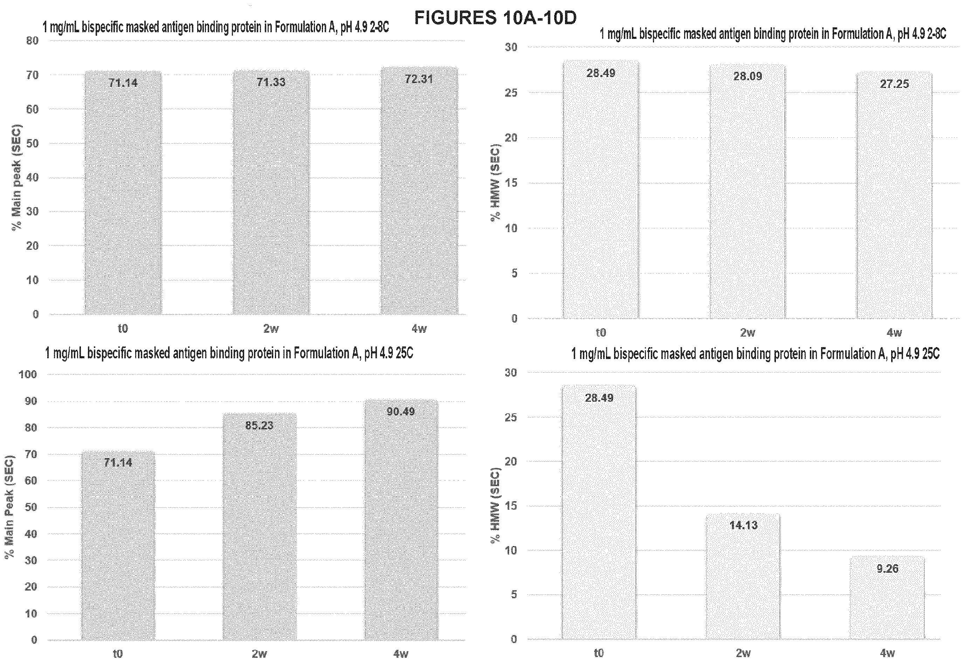

**Figures 10A-10D** are bar graphs providing the results of size exclusion ultra-high performance liquid chromatography (SE-UHPLC) analysis, showing the % Main Peak (left panels) and % high molecular weight species (right panels) of the masked bispecific antibody in Formulation A, when stored at either 2-8°C or 25°C at 0, 2 weeks and 4 weeks.

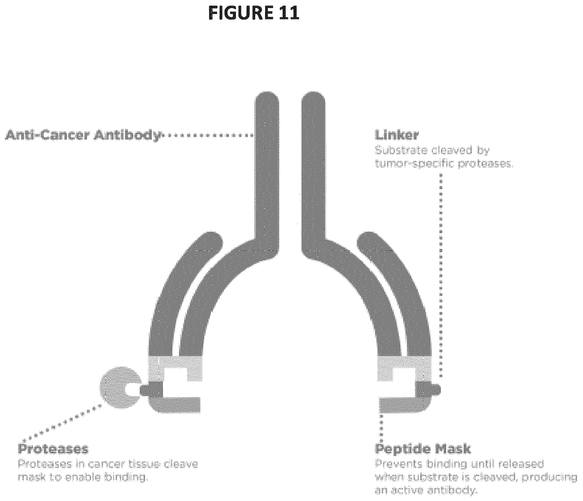

**Figure 11** is an illustration of an exemplary masked antigen binding protein also known as a "Probody."

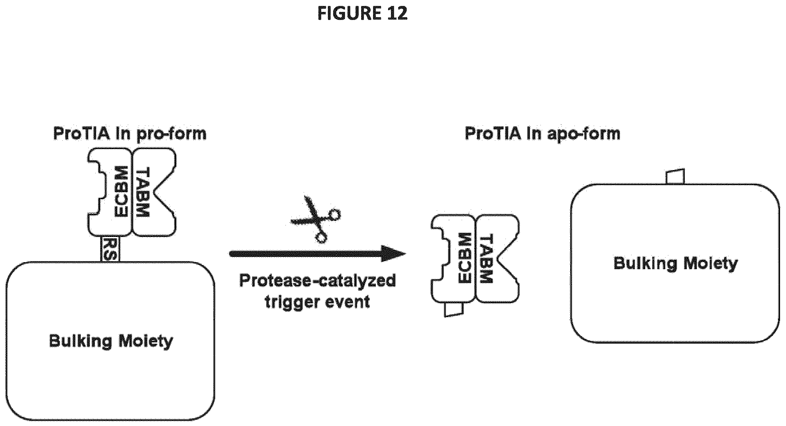

**Figure 12** is an illustration of an exemplary masked antigen binding protein also known as a "ProTIA prodrug." ECBM" is an antigen binding domain (i.e. an antigen binding domain (AB1)); "TABM" is a second antigen binding domain (i.e. a second antigen binding domain (AB2)); "RS" is a peptide or polypeptide sequence that can be cleaved by a protease (i.e. a protein recognition domain (PR)); "Bulking Moiety" is a peptide or polypeptide that inhibits or reduces the ability of the ECMB and/or TABM to bind to an antigen (e.g., a masking domain (MD)). Once the ProTIA in pro-form is cleaved at the RS site, the ECBM and/or TABM binding to antigen is increased.

### DETAILED DESCRIPTION

Specific protein-based pharmaceuticals, including antibodies, are generally not stable in liquid formulations over a long period of time and especially not at accelerated temperatures, e.g., 25°C and above. The present disclosure describes low pH formulations comprising a masked antigen binding protein that binds one, two or more antigens. In some embodiments, the bispecific or multispecific masked antigen binding protein specifically binds CD3 (including, e.g., the epsilon chain of CD3) and a cell-surface antigen (including, e.g., EGFR) in such a manner to transiently bring malignant cells within proximity to activated T cells, thereby inducing T cell mediated killing of the antibody-bound malignant cell.

A general concept underlying the present invention is the discovery that colloidal stability of a liquid pharmaceutical composition comprising a masked antigen binding protein (including, e.g., bispecific or multispecific masked antigen binding proteins) is significantly improved at a pH that is both (1) lower than the isoelectric point (pI) of the antibody or antigen binding domain of the masked antigen binding protein (e.g., VH/VL regions or antigen binding fragments thereof or AB1 and/or AB2 region as described below) and (2) lower than the pI of the masking domains (MD1 and MD2 regions, described further below) of the masked antigen binding protein. The heavy and light chains of a masked antigen binding protein typically have different isoelectric points (pI), with the complete heavy chain generally having a higher pI than the complete light chain. The pI of a masked antigen binding protein (including, e.g., bispecific or multispecific masked antigen binding proteins) and/or regions or domains within the masked antigen binding protein can be determined by any of a number of means (including, e.g., using the Bjellqvist algorithm as described in Perez-Riverol, Y. et al. Isoelectric point optimization using peptide descriptors and support vector machines. Journal of proteomics 75, 2269-2274 (2012), herein incorporated by reference). *See also*, Kozlowski LP. IPC - Isoelectric Point Calculator. Biology Direct. 2016;11:55, herein incorporated by reference, describing details of Isoelectric Point Calculator (IPC) available online at http://isoelectric.ovh.org.

The pI of the region of the masked antigen binding protein (MD1 and/or MD2 regions, as described further below) is often different than the pI of the antigen binding portions of the masked antigen binding protein (including, e.g., the heavy/light chain or antigen binding thereof, or AB1 and/or AB2 regions as described below).

The pI of other representative, theoretical AB and MD regions of theoretical masked antigen binding proteins are provided in Table 1. The recommended pH to reduce aggregation and improve stability for these representative, theoretical masked antigen binding proteins, bispecific masked antigen binding proteins and multispecific masked antigen binding proteins is also provided in Table 1.

**TABLE 1**

| **Antibody** | **AB1 pI** | **AB2 pI** | **AB3 pI** | **MD1 pI** | **MD2 pI** | **MD3 pI** | **pH** |
|---|---|---|---|---|---|---|---|
| Masked antigen binding protein 1 | 8.24 | -- | -- | 5.7 | | -- | Less than 5.7 or 3.5-5.7 |
| Masked antigen binding protein 2 | 6.57 | -- | -- | 6.63 | -- | -- | Less than 6.57 or 3.5-6.57 |
| ***Bispecific*** | | | | | | | |
| Masked antigen binding protein 1 | 8.34 | 6.29 | - | 5.5 | 6.74 | -- | Less than 5.5 or 3.5-5.5 |
| ***Bispecific*** | | | | | | | |
| Masked antigen binding protein 2 | 8.24 | 6.28 | | 4.74 | 6.53 | | Less than 4.74 or 3.5-4.74 |
| ***Multispecific*** | | | | | | | |
| Masked antigen binding protein 1 | 8.34 | 6.29 | 6.84 | 5.5 | 6.75 | 4.3 | Less than 4.3 or 3.5-4.3 |
| ***Multispecific*** | | | | | | | |
| Masked antigen binding protein 2 | 8.52 | 6.54 | 7.22 | 6.61 | 6.52 | 6.8 | Less than 6.52 or 3.5-6.52 |

Accordingly, herein we describe pharmaceutical compositions where the pH of the masked antigen binding protein composition is (1) lower than the isoelectric point (pI) of the antibody or antigen binding domains of the masked antigen binding protein (e.g., the VH/VL regions or antigen binding fragments thereof or AB1 and/or AB2 regions, as described further below) and/or (2) lower than the pI of the masking domain (MD1 and MD2 regions, as described further below) of the masked antigen binding protein. While not wishing to be bound to any particular theory, the low pH of the pharmaceutical composition ensures that sub-regions (including, e.g., the antigen binding region and the masking regions or AB1 and/or AB2 regions, as described further below) of the masked antigen binding protein have a net positive charge, which results in both inter- and intra- domain repulsion which reduces antibody aggregation and increases stability. In instances where a domain (e.g., the masking domain) has an acidic pI and another domain (e.g., the antibody or antigen binding domain) has a basic pI, a dipole may result if the pH of the pharmaceutical composition is between the pI of each of the domains (e.g., at pH 7), leading to intra- and intermolecular electrostatic attractions which increase antibody aggregation and reduce stability. While not wishing to be bound to any particular theory, the electrostatic attractions likely lead to aggregation and, thus, to the formation of undesired high molecular weight (HMW) species. The HMW species crucially impact the stability of the solution or the colloidal stability of the dispersion. This is a particular issue with masked antigen binding proteins (including bispecific or multispecific masked antigen binding proteins), which may have multiple antigen binding domains and/or multiple masking polypeptides, with each masking polypeptide interacting locally with surrounding amino acids resulting in further complications. The low pH of the composition of the disclosure allows protonation of various domains and electrostatic repulsion, thereby resulting in an unexpectedly reduced incidence of HMW species of masked antigen binding proteins (including, e.g., bispecific or multispecific antigen binding proteins). In some embodiments, the pH of the pharmaceutical composition ranges from 3.5 to 5.5. Preferred is a pH value of 4.0 to 5.5, such as a pH value of 4.2 to 4.8. In some embodiments, a preferred pH value is a pH value of 3.6 to 4.2. In some embodiments, the pH of the composition is about 2 pH units lower than the pI of AB1, AB2, MD1, and/or MD2 region(s) of the masked antigen binding protein (described further below). In some embodiments, the pH of the composition is between about 7.0 to about 3.6. In some embodiments, the pH of the composition is greater than about 3.5.

Various aspects of the formulation are described below. The use of section headings are merely for the convenience of reading, and not intended to be limiting per se. The entire document is intended to be viewed as a unified disclosure, and it should be understood that all combinations of features described herein are contemplated.

### Buffers

The pharmaceutical composition of the disclosure comprises a buffer, which optionally may be selected from the group consisting of acetate, glutamate, citrate, succinate, tartrate, fumarate, maleate, histidine, phosphate, 2-(N-morpholino)ethanesulfonate, potassium phosphate, potassium salts, a divalent cation, calcium, magnesium, manganese, zinc, acetic acid/sodium acetate, citric acid/sodium citrate, succinic acid/sodium succinate, tartaric acid/sodium tartrate, histidine/histidine HCl, glycine, Tris, glutamate, an amino acid, lysine, arginine, proline, and combinations thereof. In some embodiments, the pharmaceutical composition comprises at least one buffer selected from the group consisting of acetate, glutamate, citrate, succinate, tartrate, fumarate, maleate, histidine, phosphate, 2-(N-morpholino)ethanesulfonate and combinations thereof.

Buffering agents are often employed to control pH in the formulation. In some embodiments, the buffer is added in a concentration that maintains pH of the formulation at about 3.5 to about 5, or about 4.0 to about 5.5, or about 4.0 to about 5.0, or about 4.2 to about 4.8, or 4.2, or 3.6, or greater than 3.5. The effect of pH on formulations may be characterized using any one or more of several approaches such as accelerated stability studies.

Organic acids, phosphates and Tris are suitable buffers in protein formulations (Table 2). The buffer capacity of the buffering species is maximal at a pH equal to the pKa and decreases as pH increases or decreases away from this value. Ninety percent of the buffering capacity exists within one pH unit of its pKa. Buffer capacity also increases proportionally with increasing buffer concentration.

Several factors are typically considered when choosing a buffer. For example, the buffer species and its concentration should be defined based on its pKa and the desired formulation pH. Also important is to ensure that the buffer is compatible with the protein drug, other formulation excipients, and does not catalyze any degradation reactions. Recently, polyanionic carboxylate buffers such as citrate and succinate have been shown to form covalent adducts with the side chain residues of proteins. A third aspect to be considered is the sensation of stinging and irritation the buffer may induce.

**TABLE 2: Commonly used buffering agents and their pKₐ values**

| **Buffer** | **pKₐ** | **Example drug product** |
|---|---|---|
| Acetate | 4.8 | Neupogen, Neulasta |
| Succinate | pKₐ₁ = 4.8, pKₐ₂ = 5.5 | Actimmune |
| Citrate | pKₐ₁ = 3.1, pKₐ₂ = 4.8, pKₐ₃ = 6.4 | Humira |
| Histidine (imidazole) | 6.0 | Xolair |
| Phosphate | pKₐ₁ = 2.15, pKₐ₂ = 7.2, pKₐ₃ = 12.3 | Enbrel (liquid formulation) |
| Tris | 8.1 | Leukine |

The buffer system present in the formulation is selected to be physiologically compatible and to maintain a desired pH.

The buffer may be present in any amount suitable to maintain the pH of the formulation at a predetermined level. The buffer may be present at a concentration between about 0.1 mM and about 1000 mM (1 M), or between about 5 mM and about 200 mM, or between about 5 mM to about 100 mM, or between about 10 mM and 50 about mM. Suitable buffer concentrations encompass concentrations of about 200 mM or less. In some embodiments, the buffer in the formulation is present in a concentration of about 190 mM, about 180 mM, about 170 mM, about 160 mM, about 150 mM, about 140 mM, about 130 mM, about 120 mM, about 110 mM, about 100 mM, about 80 mM, about 70 mM, about 60 mM, about 50 mM, about 40 mM, about 30 mM, about 20 mM, about 10 mM or about 5 mM. In some embodiments, the concentration of the buffer is at least about 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, 90, 100, 200, 500, 700, or 900 mM. In some embodiments, the concentration of the buffer is between about 1, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, or 90 mM and 100 mM. In some embodiments, the concentration of the buffer is between about 5, 6, 7, 8, 9, 10, 11, 12, 13, 14, 15, 16, 17, 18, 19, 20, 30, or 40 mM and 50 mM. In some embodiments, the concentration of the buffer is about 10 mM.

Other exemplary pH buffering agents used to buffer the formulation as set out herein include, but are not limited to glycine, glutamate, succinate, phosphate, acetate, and aspartate. Amino acids such as histidine, arginine, proline, and glutamic acid can also be used as buffering agents.

### Polyols

The pharmaceutical compositions described herein also comprise at least one polyol. Polyols encompass a class of excipients that includes sugars (e.g., mannitol, sucrose, or sorbitol), and other polyhydric alcohols (e.g., glycerol and propylene glycol). The polymer polyethylene glycol (PEG) is included in this category. Polyols are commonly used as stabilizing excipients and/or isotonicity agents in both liquid and lyophilized parenteral protein formulations. Polyols can protect proteins from both physical and chemical degradation pathways.

Exemplary polyols suitable for the pharmaceutical compositions disclosed herein include, but are not limited to, sucrose, trehalose, mannose, maltose, lactose, glucose, raffinose, cellobiose, gentiobiose, isomaltose, arabinose, glucosamine, fructose, mannitol, sorbitol, glycine, arginine HCL, poly-hydroxy compounds (including, e.g., polysaccharides such as dextran, starch, hydroxyethyl starch, cyclodextrins, captisol, N-methyl pyrollidene, cellulose and hyaluronic acid), and sodium chloride (Carpenter et al., Develop. Biol. Standard 74:225, (1991)).

Additional polyols suitable for the pharmaceutical compositions disclosed herein include, but are not limited to, propylene glycol, glycerin (glycerol), threose, threitol, erythrose, erythritol, ribose, arabinose, arabitol, lyxose, maltitol, sorbitol, sorbose, glucose, mannose, mannitol, levulose, dextrose, maltose, trehalose, fructose, xylitol, inositol, galactose, xylose, fructose, sucrose, 1,2,6-hexanetriol and the like. Higher order sugars include dextran, propylene glycol, or polyethylene glycol. Reducing sugars such as fructose, maltose or galactose oxidize more readily than do non-reducing sugars. Additional examples of sugar alcohols are glucitol, maltitol, lactitol or iso-maltulose. Examples of reducing sugars include glucose, maltose, lactose, maltulose, iso-maltulose and lactulose. Examples of non-reducing sugars include non-reducing glycosides of polyhydroxy compounds selected from sugar alcohols and other straight chain polyalcohols. Monoglycosides include compounds obtained by reduction of disaccharides such as lactose, maltose, lactulose and maltulose.

In some embodiments, the at least one polyol is selected from the group consisting of a monosaccharide, a disaccharide, a cyclic polysaccharide, a sugar alcohol, a linear branched dextran, and a linear non-branched dextran, or combinations thereof. In some embodiments, the at least one polyol is a disaccharide selected from the group consisting of sucrose, trehalose, mannitol, and sorbitol or a combination thereof.

In some embodiments, the pharmaceutical composition comprises at least one polyol (e.g., saccharide) at a concentration of about 0% to about 40% w/v, or about 0% to about 20% w/v, or about 1% to about 15% w/v. In some embodiments, the pharmaceutical composition comprises at least one polyol (e.g., saccharide) at a concentration of at least 0.5, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 30, or at least 40% w/v. In some embodiments, the pharmaceutical composition comprises at least one polyol (e.g., saccharide) at a concentration of about 1, about 2, about 3, about 4, about 5, about 6, about 7, about 8, about 9, about 10, about 11, about 12, about 13, about 14% to about 15% w/v. In some embodiments, the pharmaceutical composition comprises at least one polyol (e.g., saccharide) at a concentration of about 1% to about 15% w/v. In a yet further embodiment, the pharmaceutical composition comprises at least one polyol (e.g., saccharide) at a concentration of about 9%, about 9.5%, about 10%, about 10.5%, about 11%, about 11.5%, or about 12% w/v. In some embodiments, the pharmaceutical composition comprises at least one polyol (e.g., saccharide) at a concentration of about 9% to about 12% w/v. In some embodiments, the at least one polyol (e.g., saccharide) is in the composition at a concentration of about 9% w/v. In some embodiments, the at least one polyol is selected from the group consisting of sucrose, trehalose, mannitol and sorbitol or a combination thereof. In some embodiments, the polyol is sucrose and is present in the composition at a concentration ranging from about 9% to about 12% w/v.

If desired, the formulation may also include appropriate amounts of bulking and osmolarity regulating agents, such as a saccharide, suitable for forming a lyophilized "cake."

In a preferred embodiment, the masked antigen binding protein pharmaceutical composition or formulation comprises 10 mM glutamate or 10 mM acetate, 9% (w/V) sucrose and 0.01% (w/V) polysorbate 80, wherein the pH of the pharmaceutical composition or formulation is 4.2 or 3.6.

### Surfactants

The pharmaceutical compositions disclosed herein may further optionally comprise at least one surfactant. Surfactants are commonly used in protein formulations to prevent surface-induced degradation. Surfactants are amphipathic molecules with the capability of out-competing proteins for interfacial positions. Hydrophobic portions of the surfactant molecules occupy interfacial positions (e.g., air/liquid), while hydrophilic portions of the molecules remain oriented towards the bulk solvent. At sufficient concentrations (typically around the detergent's critical micellar concentration), a surface layer of surfactant molecules serves to prevent protein molecules from adsorbing at the interface. Surface-induced degradation is thereby minimized. Surfactants include, e.g., fatty acid esters of sorbitan polyethoxylates, such as polysorbate 20 and polysorbate 80 (see e.g., Avonex^{®}, Neupogen^{®}, Neulasta^{®}). The two differ only in the length of the aliphatic chain that imparts hydrophobic character to the molecules, C-12 and C-18, respectively. Accordingly, polysorbate-80 is more surface-active and has a lower critical micellar concentration than polysorbate-20. The surfactant poloxamer 188 has also been used in several marketed liquid products such Gonal-F^{®}, Norditropin^{®}, and Ovidrel^{®}.

Detergents can also affect the thermodynamic conformational stability of proteins. Detergent destabilization of proteins can be rationalized in terms of the hydrophobic tails of the detergent molecules that can engage in specific binding with partially or wholly unfolded protein states. These types of interactions could cause a shift in the conformational equilibrium towards the more expanded protein states (i.e., increasing the exposure of hydrophobic portions of the protein molecule in complement to binding polysorbate). Alternatively, if the protein native state exhibits some hydrophobic surfaces, detergent binding to the native state may stabilize that conformation.

Another aspect of polysorbates is that they are inherently susceptible to oxidative degradation. Often, as raw materials, they contain sufficient quantities of peroxides to cause oxidation of protein residue side-chains, especially methionine. The potential for oxidative damage arising from the addition of stabilizer emphasizes the point that the lowest effective concentrations of excipients should be used in formulations. For surfactants, the effective concentration for a given protein will depend on the mechanism of stabilization. It has been postulated that if the mechanism of surfactant stabilization is related to preventing surface-denaturation the effective concentration will be around the detergent's critical micellar concentration. Conversely, if the mechanism of stabilization is associated with specific protein-detergent interactions, the effective surfactant concentration will be related to the protein concentration and the stoichiometry of the interaction (Randolph T.W., et al., Pharm Biotechnol., 13:159-75 (2002)).

Surfactants may also be added in appropriate amounts to prevent surface related aggregation phenomenon during freezing and drying (Chang, B, J. Pharm. Sci. 85:1325, (1996)). Exemplary surfactants include, but are not limited to, anionic, cationic, nonionic, zwitterionic, and amphoteric surfactants, including surfactants derived from naturally-occurring amino acids. Anionic surfactants include, but are not limited to, sodium lauryl sulfate, dioctyl sodium sulfosuccinate and dioctyl sodium sulfonate, chenodeoxycholic acid, N-lauroylsarcosine sodium salt, lithium dodecyl sulfate, 1-octanesulfonic acid sodium salt, sodium cholate hydrate, sodium deoxycholate, and glycodeoxycholic acid sodium salt. Cationic surfactants include, but are not limited to, benzalkonium chloride or benzethonium chloride, cetylpyridinium chloride monohydrate, and hexadecyltrimethylammonium bromide. Zwitterionic surfactants include, but are not limited to, CHAPS, CHAPSO, SB3-10, and SB3-12. Non-ionic surfactants include, but are not limited to, digitonin, Triton X-100, Triton X-114, TWEEN-20, and TWEEN-80. In another embodiment, surfactants include lauromacrogol 400; polyoxyl 40 stearate; polyoxyethylene hydrogenated castor oil 10, 40, 50 and 60; glycerol monostearate, polysorbate 20, 40, 60, 65 and 80; soy lecithin and other phospholipids such as DOPC, DMPG, DMPC, and DOPG; sucrose fatty acid ester; methyl cellulose and carboxymethyl cellulose.

Pharmaceutical compositions described herein may further optionally comprise at least one surfactant, either individually or as a mixture in different ratios. In some embodiments, the composition comprises a surfactant at a concentration of about 0.001% to about 5% w/v. In some embodiments, the composition comprises a surfactant at a concentration of at least 0.001, at least 0.002, at least 0.003, at least 0.004, at least 0.005, at least 0.007, at least 0.01, at least 0.05, at least 0.1, at least 0.2, at least 0.3, at least 0.4, at least 0.5, at least 0.6, at least 0.7, at least 0.8, at least 0.9, at least 1.0, at least 1.5, at least 2.0, at least 2.5, at least 3.0, at least 3.5, at least 4.0, or at least 4.5% w/v. In some embodiments, the composition comprises a surfactant at a concentration of about 0.004% to about 0.5% w/v. In some embodiments, the composition comprises a surfactant at a concentration of about 0.004, about 0.005, about 0.007, about 0.01, about 0.05, about 0.1, about 0.2, about 0.3, or about 0.4% w/v to about 0.5% w/v. In some embodiments, the composition comprises a surfactant that is incorporated in a concentration of about 0.001% to about 0.01% w/v. In some embodiments, the surfactant is polysorbate 80, and the polysorbate 80 is present in a concentration of about 0.01% w/v.

### Other Considerations

As used herein, the term "pharmaceutical composition" relates to a composition which is suitable for administration to a subject in need thereof. The terms "subject" or "individual" or "animal" or "patient" are used interchangeably herein to refer to any subject, particularly a mammalian subject, for whom administration of the pharmaceutical composition is desired. Mammalian subjects include humans, non-human primates, dogs, cats, guinea pigs, rabbits, rats, mice, horses, cattle, cows, and the like, with humans being preferred. The pharmaceutical composition of the present disclosure is stable and pharmaceutically acceptable, i.e., capable of eliciting the desired therapeutic effect without causing any undesirable local or systemic effects in the subject to which the pharmaceutical composition is administered. Pharmaceutically acceptable compositions of the invention may in particular be sterile and/or pharmaceutically inert. Specifically, the term "pharmaceutically acceptable" can mean approved by a regulatory agency or other generally recognized pharmacopoeia for use in animals, and more particularly in humans.

The pharmaceutical compositions provided by the disclosure comprises a masked antigen binding protein (e.g., a bispecific or multispecific masked antigen binding protein). In some embodiments, the masked antigen binding protein is provided in a therapeutically effective amount. By "therapeutically effective amount" is meant an amount of said masked antigen binding protein (including e.g., bispecific or multispecific masked antigen binding proteins) that elicits the desired therapeutic effect. Therapeutic efficacy and toxicity can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, ED50/LD50. Pharmaceutical compositions that exhibit large therapeutic indices are generally preferred.

Protein and antibody formulations, including the pharmaceutical compositions disclosed herein, may be administered parenterally. When given parenterally, they must be sterile. Sterile diluents include liquids that are pharmaceutically acceptable (safe and non-toxic for administration to a human) and useful for the preparation of a liquid pharmaceutical composition, such as a pharmaceutical composition reconstituted after lyophilization. Exemplary diluents include sterile water, bacteriostatic water for injection (BWFI), a pH buffered solution (e.g., phosphate-buffered saline), sterile saline solution, Ringer's solution or dextrose solution. Diluents can include aqueous solutions of salts and/or buffers.

Excipients are additives that are included in a pharmaceutical composition or formulation because they either impart or enhance the stability, delivery and manufacturability of a drug product. Regardless of the reason for their inclusion, excipients must be safe and tolerated by patients. For protein drugs and antibodies, the choice of excipients is particularly important because they can affect both efficacy and immunogenicity of the drug. Hence, protein formulations need to be developed with appropriate selection of excipients that afford suitable stability, safety, and marketability.

The excipients described herein are organized either by their chemical type or their functional role in the pharmaceutical composition/formulation. Given the teachings and guidance provided herein, those skilled in the art will readily be able to vary the amount or range of excipient without increasing viscosity to an undesirable level. Excipients may be chosen to achieve a desired osmolality (i.e., isotonic, hypotonic or hypertonic) of the final solution, pH, desired stability, resistance to aggregation or degradation or precipitation, protection under conditions of freezing, lyophilization or high temperatures, or other properties. A variety of types of excipients are known in the art. Exemplary excipients include salts, amino acids, other tonicity agents, surfactants, stabilizers, bulking agents, cryoprotectants, lyoprotectants, anti-oxidants, metal ions, chelating agents and/or preservatives.

Further, where a particular excipient is reported in a pharmaceutical composition or formulation by, e.g., percent (%) w/v, those skilled in the art will recognize that the equivalent molar concentration of that excipient is also contemplated.

### Other Stabilizers and Bulking Agents

Pharmaceutical compositions described herein may further optionally comprise at least one stabilizer (including a sugar, a polymer and/or a polyol). Stabilizers include a class of compounds that can serve as cryoprotectants, lyoprotectants, and glass forming agents. Cryoprotectants act to stabilize proteins during freezing or in the frozen state at low temperatures. Lyoprotectants stabilize proteins or antibodies in the freeze-dried solid dosage form by preserving the native-like conformational properties of the protein during dehydration stages of freeze-drying. Glassy state properties have been classified as "strong" or "fragile" depending on their relaxation properties as a function of temperature. It is important that cryoprotectants, lyoprotectants, and glass forming agents remain in the same phase with the protein or antibody in order to impart stability. Exemplary lyoprotectants include, but are not limited to, glycerin and gelatin, and the sugars mellibiose, melezitose, raffinose, mannotriose and stachyose.

### Amino acids

In some embodiments, the pharmaceutical compositions described herein further comprise one or more amino acids. Amino acids have found versatile use in protein and antibody formulations or pharmaceutical compositions as buffers, bulking agents, stabilizers and antioxidants. Histidine and glutamic acid are employed to buffer protein or antibody formulations in the pH range of 5.5 - 6.5 and 4.0 - 5.5 respectively. The imidazole group of histidine has a pKa = 6.0 and the carboxyl group of glutamic acid side chain has a pKa of 4.3 which makes them suitable for buffering in their respective pH ranges. Glutamic acid is found in some formulations (e.g., Stemgen^{®}). Histidine is commonly found in marketed protein/antibody formulations (e.g., Xolair^{®}, Herceptin^{®}, Recombinate^{®}). It provides a good alternative to citrate, a buffer known to sting upon injection. Interestingly, histidine has also been reported to have a stabilizing effect when used at high concentrations in both liquid and lyophilized presentations (Chen B, et al., Pharm Res., 20(12): 1952-60 (2003)). Histidine (up to 60 mM) was also observed to reduce the viscosity of a high concentration formulation of an antibody. However, in the same study, the authors observed increased aggregation and discoloration in histidine containing formulations during freeze-thaw studies of the antibody in stainless steel containers. The authors attributed this to an effect of iron ions leached from corrosion of steel containers. Another note of caution with histidine is that it undergoes photooxidation in the presence of metal ions (Tomita M, et al., Biochemistry, 8(12): 5149-60 (1969)). The use of methionine as an antioxidant in formulations appears promising; it has been observed to be effective against a number of oxidative stresses (Lam XM, et al., J Pharm Sci., 86(11): 1250-5 (1997)).

The amino acids glycine, proline, serine and alanine stabilize proteins or antibodies. Glycine is also a bulking agent in lyophilized formulations (e.g., Neumega^{®}, Genotropin^{®}, Humatrope^{®}). Arginine has been shown to be an effective agent in inhibiting aggregation and has been used in both liquid and lyophilized formulations (e.g., Activase^{®}, Avonex^{®}, Enbrel^{®} liquid). The pharmaceutical compositions described herein may further optionally comprise any one or more of the aforementioned amino acids.

### Antioxidants

In some embodiments, the pharmaceutical composition described herein further comprises one or more antioxidants. Oxidation of protein residues arises from a number of different sources. Beyond the addition of specific antioxidants, the prevention of oxidative protein damage involves the careful control of a number of factors throughout the manufacturing process and storage of the product such as atmospheric oxygen, temperature, light exposure, and chemical contamination. The most commonly used pharmaceutical antioxidants are reducing agents, oxygen/free-radical scavengers, or chelating agents. Antioxidants in therapeutic protein or antibody formulations must be water-soluble and remain active throughout the product shelf-life. Reducing agents and oxygen/free-radical scavengers work by ablating active oxygen species in solution. Chelating agents such as EDTA can be effective by binding trace metal contaminants that promote free-radical formation. For example, EDTA was utilized in the liquid formulation of acidic fibroblast growth factor to inhibit the metal ion catalyzed oxidation of cysteine residues. EDTA has been used in marketed products like Kineret^{®} and Ontak^{®}. Accordingly, the pharmaceutical compositions described herein may further optionally comprise any one or more of these reducing agents, oxygen/free-radical scavengers, and/or chelating agents.

However, antioxidants themselves can induce other covalent or physical changes to the protein. A number of such cases have been reported in the literature. Reducing agents (like glutathione) can cause disruption of intramolecular disulfide linkages, which can lead to disulfide shuffling. In the presence of transition metal ions, ascorbic acid and EDTA have been shown to promote methionine oxidation in a number of proteins and peptides (Akers MJ, and Defelippis MR. Peptides and Proteins as Parenteral Solutions. In: Pharmaceutical Formulation Development of Peptides and Proteins. Sven Frokjaer, Lars Hovgaard, editors. Pharmaceutical Science. Taylor and Francis, UK (1999)); Fransson J.R., J. Pharm. Sci. 86(9): 4046-1050 (1997); Yin J, et al., Pharm Res., 21(12): 2377-83 (2004)). Sodium thiosulfate has been reported to reduce the levels of light and temperature induced methionine-oxidation in rhuMab HER2; however, the formation of a thiosulfate-protein adduct was also reported in this study (Lam XM, Yang JY, et al., J Pharm Sci. 86(11): 1250-5 (1997)). Selection of an appropriate antioxidant is made according to the specific stresses and sensitivities of the masked antigen binding protein.

### Metal Ions

In some embodiments, the pharmaceutical composition further comprises one or more metal ions. In general, transition metal ions are undesired in protein or antibody pharmaceutical compositions or formulations because they can catalyze physical and chemical degradation reactions in proteins. However, specific metal ions are included in formulations when they are co-factors to proteins where they form coordination complexes (e.g., zinc suspension of insulin). Recently, the use of magnesium ions (10 -120 mM) has been proposed to inhibit the isomerization of aspartic acid to isoaspartic acid (see, e.g., International Patent Publication No. WO 2004/039337). Exemplary metal ions include, but are not limited to, Ca⁺² ions, Mg⁺², Mn⁺², Zn⁺², Cu⁺² and Fe⁺².

### Preservatives

In some embodiments, the pharmaceutical composition further comprises one or more preservatives. Preservatives are necessary when developing multi-use parenteral formulations that involve more than one extraction from the same container. Their primary function is to inhibit microbial growth and ensure product sterility throughout the shelf-life or term of use of the drug product. Commonly used preservatives include phenol, benzyl alcohol, meta-cresol, alkyl parabens such as methyl paraben or propyl paraben, benzalkonium chloride, and benzethonium chloride. Other examples of compounds with antimicrobial preservative activity include octadecyldimethylbenzyl ammonium chloride and hexamethonium chloride. Other types of preservatives include aromatic alcohols such as butyl alcohol, phenol, benzyl alcohol, atechol, resorcinol, cyclohexanol, and 3-pentanol.

Multi-use injection pen presentations optionally include preserved formulations. For example, preserved formulations of hGH are currently available on the market. Norditropin^{®} (liquid, Novo Nordisk), Nutropin AQ^{®} (liquid, Genentech) & Genotropin (lyophilized - dual chamber cartridge, Pharmacia & Upjohn) contain phenol while Somatrope^{®} (Eli Lilly) is formulated with m-cresol.

Several aspects are considered during the formulation development of preserved dosage forms. Optimization of preservative concentration involves testing a given preservative in the dosage form with concentration ranges that confer anti-microbial effectiveness without compromising protein stability. For example, three preservatives were successfully screened in the development of a liquid formulation for interleukin-1 receptor (Type I), using differential scanning calorimetry (DSC). The preservatives were rank ordered based on their impact on stability at concentrations commonly used in marketed products (Remmele RL Jr., et al., Pharm Res., 15(2): 200-8 (1998)).

Some preservatives can cause injection site reactions, which is another factor for consideration when choosing a preservative. In clinical trials that focused on the evaluation of preservatives and buffers in Norditropin, pain perception was observed to be lower in formulations containing phenol and benzyl alcohol as compared to a formulation containing m-cresol (Kappelgaard A.M., Horm Res. 62 Suppl 3:98-103 (2004)). Interestingly, among the commonly used preservative, benzyl alcohol possesses anesthetic properties (Minogue SC, and Sun DA., Anesth Analg., 100(3): 683-6 (2005)).

The disclosure also contemplates a pharmaceutical composition that does not comprise any preservatives.

### Masked Antigen-Binding Proteins

The pharmaceutical compositions or formulations described herein comprise a masked antigen binding protein. A "masked antigen binding protein" is a protein that includes a masking domain (MD) coupled (e.g., through covalent bond or a linker) to an antigen binding domain (AB) such that coupling of the MD inhibits or reduces the binding of the AB to its antigen. The MD further comprises a protein recognition site (PR) that includes a substrate or binding site for a protein or protease, such that when the protein or protease binds to and/or cleaves the protein recognition site the AB domain binds antigen or the binding to the antigen by the AB domain is increased or induced. Masked antigen binding proteins have been previously described in, e.g., International Publication No. WO 2017/040344, U.S. Patent No. 9540440, U.S. Publication No. 20150118254, U.S. Patent No. 9127053, U.S. Patent No. 9517276, and U.S. Patent No. 8563269, each of which is incorporated herein by reference in its entirety. In some embodiments, the masked antigen binding protein is a Probody (as described in, for example, Figure 11; or Polu KR and Lowman HB. Expert Opin Biol Ther. 2014 Aug;14(8):1049-53; or Desnoyers LR et. al., Sci Transl Med. 2013 Oct 16;5(207)) or a ProTIA prodrug (as described in, for example, Figure 12; or Schellenberger V. Amunix unveils next-generation immuno-oncologic cancer therapy platform. https://biopharmadealmakers.nature.com. September 2016 edition, B20; see also http://www.amunix.com/technology/pro-tia/). In some embodiments, the masked antigen binding protein has the structural arrangement from N-terminus to C-terminus as follows: MD-AB or AB-MD. In some embodiments, the masked antigen binding protein includes a linking peptide (LP), and the masked antigen binding protein has the structural arrangement from N-terminus to C-terminus as follows: MD-LP-AB or AB-LP-MD.

As discussed above, the term "masked antigen binding proteins" refers to a masked antigen binding protein in both its naive state, as well as the state when a protein or protease binds to the binding site or substrate within the protein recognition site (PR) of the masked antigen binding protein which may result in the cleavage of the PR or induce a structural confirmation change (i.e., the active state). Accordingly, it will be apparent to the skilled artisan that in some embodiments a masked antigen binding protein can lack a MD due to cleavage of the PR by a protease, resulting in release of at least the MD (e.g., where the MD is not joined to the masked antigen binding protein by a covalent bond (e.g., a disulfide bond between cysteine residues)).

When the masked antigen binding protein is in the presence of the antigen in a naive state, binding of the AB to the antigen is reduced or inhibited, as compared to the binding of the AB to the antigen when the masked antigen binding protein is in the active state (i.e., when a protein or protease binds to the PR and/or removes or dislocates the MD). When compared to the binding of the AB not associated with an MD (i.e., when the masked antigen binding protein is in the active state), the ability of the masked antigen binding protein to bind antigen in the naive state is reduced, for example, by at least about 30%, about 35%, about 40%, about 45%, about 50%, about 60%, about 70%, about 80%, about 90%, about 92%, about 93%, about 94%, about 95%, about 96%, about 97%, about 98%, about 99%, or even about 100% when measured *in vitro* and/or *in vivo* binding assays.

In some embodiments, the protein recognition site (PR) functions as a substrate for a protease, preferably an extracellular protease. The PR may be selected based on a protein or protease that is produced by a cell (including, e.g., tumor cells) in proximity to cells that express the antigen and/or produced by a cell (including, e.g., tumor cells) that is co-localized in tissue with the desired antigen of the AB of the masked antigen binding protein. In some embodiments, the protease is u-type plasminogen activator (uPA, also referred to as urokinase), legumain, and/or matriptase (also referred to as MT-SP1 or MTSP1). In some embodiments, the protease is a matrix metalloprotease (MMP). In some embodiments, the protease is one of the proteases described in Rawlings, N. and Salvesen, G. Handbook of Proteolytic Enzymes (Third Edition). Elsevier, 2013. ISBN: 978-0-12-382219-2, herein incorporated by reference for all purposes.

The pharmaceutical compositions described herein may contain a masked antigen binding protein having ABs or MDs with pIs that are acidic (less than pH 7.0), neutral (about pH 7.0) or basic (greater than pH 7.0). In some embodiments, the AB domains in a bispecific or multispecific antigen binding protein may have acidic, neutral and/or basic pIs, while the MDs may also have acidic, neutral and/or basics pIs. In some embodiments, the pharmaceutical compositions described herein contain a masked antigen binding protein having ABs and MDs with pIs that are both acidic, neutral or basic, or have an AB with an acidic pI and an MD with a neutral or basic pI. In other embodiments, the AB may have a neutral or basic pI while an MD has an acidic pI.

In some embodiments, the masked antigen binding protein includes an antibody or antigen binding fragment thereof (AB) that specifically binds an antigen. In some embodiments, the antibody or antigen binding fragment thereof that binds an antigen is a monoclonal antibody, domain antibody, a single chain antibody, a Fab fragment, a F(ab')₂ fragment, a scFv, a scAb, a dAb, a single domain heavy chain antibody, or a single domain light chain antibody. In some embodiments, the antibody or antigen binding fragment thereof is a mouse, chimeric, humanized or fully human monoclonal antibody. In some preferred embodiments, the antigen binding fragment is a Fab fragment, a F(ab')₂ fragment, a scFv, or a scAb.

In some embodiments, the antibody or antigen binding fragment thereof binds an antigen selected from the group consisting EGFR, mesothelin, CDH3, CD3 or combinations thereof. In some embodiments, the antibody or antigen binding fragment thereof binds an antigen selected from the group consisting of a hormone, growth factor, cytokine, a cell-surface receptor, or any ligand thereof. In some embodiments, the antibody or antigen binding fragment binds an antigen selected from the group consisting of such cytokines, lymphokines, growth factors, or other hematopoietic factors include, but are not limited to: M-CSF, GM-CSF, TNF, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IFN, TNFα, TNF1, TNF2, G-CSF, Meg-CSF, GM-CSF, thrombopoietin, stem cell factor, and erythropoietin. Additional growth factors for use herein include angiogenin, bone morphogenic protein-1, bone morphogenic protein-2, bone morphogenic protein-3, bone morphogenic protein-4, bone morphogenic protein-5, bone morphogenic protein-6, bone morphogenic protein-7, bone morphogenic protein-8, bone morphogenic protein-9, bone morphogenic protein-10, bone morphogenic protein-11, bone morphogenic protein-12, bone morphogenic protein-13, bone morphogenic protein-14, bone morphogenic protein-15, bone morphogenic protein receptor IA, bone morphogenic protein receptor IB, brain derived neurotrophic factor, ciliary neutrophic factor, ciliary neutrophic factor receptor α, cytokine-induced neutrophil chemotactic factor 1, cytokine-induced neutrophil, chemotactic factor 2 α, cytokine-induced neutrophil chemotactic factor 2 β, β endothelial cell growth factor, endothelin 1, epithelial-derived neutrophil attractant, glial cell line-derived neutrophic factor receptor α 1, glial cell line-derived neutrophic factor receptor α 2, growth related protein, growth related protein α, growth related protein β, growth related protein γ, heparin binding epidermal growth factor, hepatocyte growth factor, hepatocyte growth factor receptor, insulin-like growth factor I, insulin-like growth factor receptor, insulin-like growth factor II, insulin-like growth factor binding protein, keratinocyte growth factor, leukemia inhibitory factor, leukemia inhibitory factor receptor α, nerve growth factor nerve growth factor receptor, neurotrophin-3, neurotrophin-4, pre-B cell growth stimulating factor, stem cell factor, stem cell factor receptor, transforming growth factor α, transforming growth factor β, transforming growth factor β1, transforming growth factor β1.2, transforming growth factor β2, transforming growth factor β3, transforming growth factor β5, latent transforming growth factor β1, transforming growth factor β binding protein I, transforming growth factor β binding protein II, transforming growth factor β binding protein III, tumor necrosis factor receptor type I, tumor necrosis factor receptor type II, urokinase-type plasminogen activator receptor, and chimeric proteins and biologically or immunologically active fragments thereof.

In some embodiments, the antibody or antigen binding fragment binds an antigen selected from the group consisting of p53, KRAS, NRAS, MAGEA, MAGEB, MAGEC, BAGE, GAGE, LAGE/NY-ESO1, SSX, tyrosinase, gp100/pmel17, Melan-A/MART-1, gp75/TRP1, TRP2, CEA, RAGE-1, HER2/NEU, WT1.

In exemplary aspects, the antibody or antigen binding fragment binds an antigen expressed on the surface of an immune cell. In some embodiments, masked antigen binding protein binds to a cluster of differentiation molecule selected from the group consisting of: CD1a, CD1b, CD1c, CD1d, CD2, CD3, CD4, CD5, CD6, CD7, CD8, CD9, CD10, CD11A, CD11B, CD11C, CDw12, CD13, CD14, CD15, CD15s, CD16, CDw17, CD18, CD19, CD20, CD21, CD22, CD23, CD24, CD25, CD26, CD27, CD28, CD29, CD30, CD31,CD32, CD33, CD34, CD35, CD36, CD37, CD38, CD39, CD40, CD41, CD42a, CD42b, CD42c, CD42d, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d, CD66e, CD66f, CD68, CD69, CD70, CD71, CD72, CD73, CD74, CD75, CD76, CD79α, CD79β, CD80, CD81, CD82, CD83, CDw84, CD85, CD86, CD87, CD88, CD89, CD90, CD91, CDw92, CD93, CD94, CD95, CD96, CD97, CD98, CD99, CD100, CD101, CD102, CD103, CD104, CD105, CD106, CD107a, CD107b, CDw108, CD109, CD114, CD 115, CD116, CD117, CD118, CD119, CD120a, CD120b, CD121a, CDw121b, CD122, CD123, CD124, CD125, CD126, CD127, CDw128, CD129, CD130, CDw131, CD132, CD134, CD135, CDw136, CDw137, CD138, CD139, CD140a, CD140b, CD141, CD142, CD143, CD144, CD145, CD146, CD147, CD148, CD150, CD151, CD152, CD153, CD154, CD155, CD156, CD157, CD158a, CD158b, CD161, CD162, CD163, CD164, CD165, CD 166, and CD 182.

In some embodiments, the antibody or antigen binding fragment is selected from the group consisting of Muromonab-CD3 (product marketed with the brand name Orthoclone Okt3^{®}), Abciximab (product marketed with the brand name Reopro^{®}.), Rituximab (product marketed with the brand name MabThera^{®}, Rituxan^{®}) (U.S. Patent No. 5,843,439), Basiliximab (product marketed with the brand name Simulect^{®}), Daclizumab (product marketed with the brand name Zenapax^{®}), Palivizumab (product marketed with the brand name Synagis^{®}), Infliximab (product marketed with the brand name Remicade^{®}), Trastuzumab (product marketed with the brand name Herceptin^{®}), Alemtuzumab (product marketed with the brand name MabCampath^{®}, Campath-1H^{®}), Adalimumab (product marketed with the brand name Humira^{®}), Tositumomab-I131 (product marketed with the brand name Bexxar^{®}), Efalizumab (product marketed with the brand name Raptiva^{®}), Cetuximab (product marketed with the brand name Erbitux^{®}), Ibritumomab tiuxetan (product marketed with the brand name Zevalin^{®}), Omalizumab (product marketed with the brand name Xolair^{®}), Bevacizumab (product marketed with the brand name Avastin^{®}), Natalizumab (product marketed with the brand name Tysabri^{®}), Ranibizumab (product marketed with the brand name Lucentis^{®}), Panitumumab (product marketed with the brand name Vectibix^{®}), Eculizumab (product marketed with the brand name Soliris^{®}), Certolizumab pegol (product marketed with the brand name Cimzia^{®}), Golimumab (product marketed with the brand name Simponi^{®}), Canakinumab (product marketed with the brand name Ilaris^{®}), Catumaxomab (product marketed with the brand name Removab^{®}), Ustekinumab (product marketed with the brand name Stelara^{®}), Tocilizumab (product marketed with the brand name RoActemra^{®}, Actemra^{®}), Ofatumumab (product marketed with the brand name Arzerra^{®}), Denosumab (product marketed with the brand name Prolia^{®}), Belimumab (product marketed with the brand name Benlysta^{®}), Raxibacumab, Ipilimumab (product marketed with the brand name Yervoy^{®}), and Pertuzumab (product marketed with the brand name Perjeta^{®}). In exemplary embodiments, the antibody is one of anti-TNF alpha antibodies such as adalimumab, infliximab, etanercept, golimumab, and certolizumab pegol; anti-IL1β antibodies such as canakinumab; anti-IL12/23 (p40) antibodies such as ustekinumab and briakinumab; and anti-IL2R antibodies, such as daclizumab. Examples of suitable anti-cancer antibodies include, but are not limited to, anti-BAFF antibodies such as belimumab; anti-CD20 antibodies such as rituximab; anti-CD22 antibodies such as epratuzumab; anti-CD25 antibodies such as daclizumab; anti-CD30 antibodies such as iratumumab, anti-CD33 antibodies such as gemtuzumab, anti-CD52 antibodies such as alemtuzumab; anti-CD152 antibodies such as ipilimumab; anti-EGFR antibodies such as cetuximab; anti-HER2 antibodies such as trastuzumab and pertuzumab; anti-IL6 antibodies such as siltuximab; anti-VEGF antibodies such as bevacizumab; and anti-IL6 receptor antibodies such as tocilizumab.

In some embodiments, the pharmaceutical composition comprises a bispecific or multispecific masked antigen binding protein. Bispecific or multispecific masked antigen binding proteins have been previously described. See, e.g., International Publication No. WO 2017/040344; U.S. Patent Publication No. 2015/0079088 (herein incorporated by reference). A "bispecific masked antigen binding protein" is a masked antigen binding protein that binds two different antigens or epitopes, while a "multispecific masked antigen binding protein" is a masked antigen binding protein that recognizes three or more different antigens or epitopes.

In some embodiments, one antibody or antigen binding fragment thereof (AB1) domain of a bispecific or multispecific antigen binding protein has specificity for a target antigen and another antibody or antigen binding fragment thereof (AB2) domain has specificity for another target antigen. In some embodiments, one antibody or antigen binding fragment thereof (AB1) domain of a bispecific or multispecific antigen binding protein has specificity for an epitope of a target antigen and another antibody or antigen binding fragment thereof (AB2) domain has specificity for another epitope of the same target antigen.

A bispecific or multispecific masked antigen binding protein includes at least one masking domain (MD) comprising a protein recognition site (PR), wherein the MDs inhibit or reduce the binding of AB to an antigen. The at least one MD comprises a protein recognition site (PR) that includes a substrate or binding site for a protein or protease, such that when the protein or protease binds to and/or cleaves the protease recognition site the AB domain binds antigen or binding is increased. For a bispecific masked antigen binding protein, the masked antigen binding protein preferably comprises two MDs (e.g., MD1 and MD2) that reduce the ability of each antigen binding domain (AB 1 and AB2) to bind its respective antigen or epitope.

The masked antigen binding proteins and/or bispecific or multispecific masked antigen binding proteins provided herein are stable in circulation and activated at intended sites of therapy and/or diagnosis, but not in normal (i.e., healthy) tissue. When in an active state, the masked antigen binding proteins and/or bispecific or multispecific masked antigen binding proteins exhibit binding to an antigen that is at least comparable to the corresponding, unmodified antibody or bispecific or multispecific antigen binding protein (i.e., counterpart antibody which does not comprise a masking domain).

The term "antibody" refers to a protein having a conventional immunoglobulin format, comprising heavy and light chains, and comprising variable and constant regions. For example, an antibody may be an IgG which is a "Y-shaped" structure of two identical pairs of polypeptide chains, each pair having one "light" (typically having a molecular weight of about 25 kDa) and one "heavy" chain (typically having a molecular weight of about 50-70 kDa). An antibody has a variable region and a constant region. In IgG formats, a variable region is generally about 100-110 or more amino acids, comprises three complementarity determining regions (CDRs), is primarily responsible for antigen recognition, and substantially varies among other antibodies that bind to different antigens. See, e.g., Janeway et al., "Structure of the Antibody Molecule and the Immunoglobulin Genes", Immunobiology: The Immune System in Health and Disease, 4th ed. Elsevier Science Ltd./Garland Publishing, (1999). The antibody may further optionally comprise a constant domain such that the antibody can be a IgA, IgD, IgE, IgG, or IgM antibody, including any one of IgG1, IgG2, IgG3 or IgG4. A variable region typically comprises at least three heavy or light chain CDRs (Kabat et al., 1991, Sequences of Proteins of Immunological Interest, Public Health Service N.I.H., Bethesda, Md.; see also Chothia and Lesk, 1987, J. Mol. Biol. 196:901-917; Chothia et al., 1989, Nature 342: 877-883), within a framework region (designated framework regions 1-4, FR1, FR2, FR3, and FR4, by Kabat et al., 1991; see also Chothia and Lesk, 1987, supra). The constant region allows the antibody to recruit cells and molecules of the immune system.

The term "antibody fragment", "antibody fragment thereof, or "antigen binding antibody fragment" refers to a portion of an intact antibody. An "antigen binding fragment" or "antigen binding fragment thereof' refers to a portion of an intact antibody that binds to an antigen. An antigen binding fragment can contain the antigenic determining variable regions of an intact antibody. Examples of antibody fragments antigen binding fragment include, but are not limited to Fab, Fab', F(ab')2, and Fv fragments, linear antibodies, scFvs, and single chain antibodies.

In various aspects, the antibody is a monoclonal antibody. In certain aspects, the antibody is a human antibody. In certain aspects, the antibody is chimeric or humanized. The term "chimeric" refers to an antibody containing domains from two or more different antibodies. A chimeric antibody can, for example, contain the constant domains from one species and the variable domains from a second, or more generally, can contain stretches of amino acid sequence from at least two species. Both "chimeric" and "humanized" often refer to antigen binding proteins that combine regions from more than one species. A chimeric antibody also can contain domains of two or more different antibodies within the same species. In one embodiment, the chimeric antibody is a CDR grafted antibody.

The term "humanized" when used in relation to antigen binding proteins refers to antigen binding proteins (e.g., antibodies) having at least CDR region from a non-human source and which are engineered to have a structure and immunological function more similar to true human antibodies than the original source antibodies. For example, humanizing can involve grafting a CDR from a non-human antibody, such as a mouse antibody, into a human framework region. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., International Patent Publication No. WO 92/11018; Jones, 1986, Nature 321:522-525; and Verhoeyen et al., 1988, Science 239:1534-1536, all entirely incorporated by reference. "Back mutation" of selected acceptor framework residues to the corresponding donor residues is often employed to regain affinity that is lost in the initial grafted construct (See, e.g., U.S. Patent Nos. 5530101; 5585089; 5693761; 5693762; 6180370; 5859205; 5821337; 6054297; and 6407213, all entirely incorporated by reference). The humanized antibody optimally also will comprise at least a portion of an immunoglobulin constant region, typically that of a human immunoglobulin, and thus will typically comprise a human Fc region.

Non-limiting examples of multispecific antibodies include trispecific antibodies, tetraspecific antibodies, and the like. Masked antigen binding proteins, including bispecific or multispecific masked antigen binding proteins, are optionally multivalent; multivalency refers to the total number of binding sites on the antibody, regardless of whether the binding sites recognize the same or different antigens or epitopes.

The bispecific or multispecific masked antigen binding proteins described herein, in various embodiments, bind human CD3 (SEQ ID NO: 3). For example, in various aspects, the bispecific or multispecific masked antigen binding proteins activate T cells via engagement of CD3ε on the T cells. That is, the antibodies agonize, stimulate, activate, and/or augment CD3-mediated T cell activation. Biological activities of CD3 include, for example, T cell activation and other signaling through interaction between CD3 and the antigen binding subunits of the T-Cell Receptor (TCR).

The bispecific or multispecific masked antigen binding proteins disclosed herein optionally bind to CD3ε with a binding constant (Kd) of ≦1 µM, for example, in some embodiments, ≦100 nM, ^ 10 nM, or ≦1 nM.

In various aspects, the masked antigen binding protein (e.g., bispecific or multispecific masked antigen binding protein) binds epidermal growth factor receptor (EGFR). The sequence of human EGFR is provided as SEQ ID NO: 4. The multispecific masked antigen binding proteins disclosed herein optionally bind to human EGFR with a binding constant (Kd) of ^ 1 µM, for example, in some embodiments, ≦100 nM, ^ 10 nM, or ^ 1 nM.

In preferred embodiments, the masked bispecific or multispecific antigen binding protein binds both CD3 and EGFR.

In some embodiments, the masked antigen binding protein is heterodimeric such that it includes an antigen binding domain that is a Fab (e.g., an IgG Fab) and an antigen binding domain that is a scFv. For example, in an exemplary embodiment, the masked antigen binding protein comprises a heavy and light chain (e.g., IgG heavy and light chain) that binds one target (e.g., EGFR) and a scFv domain that binds a second target (e.g., a T-cell surface antigen such as CD3). A single-chain antibody (scFv) is an antigen binding protein in which a VL and a VH region are joined via a linker (e.g., a synthetic sequence of amino acid residues usually about 15 to about 20 amino acids in length) to form a continuous protein chain wherein the linker is long enough to allow the protein chain to fold back on itself and form a monovalent antigen binding site (see, e.g., Bird et al., 1988, Science 242:423-26 and Huston et al., 1988, Proc. Natl. Acad. Sci. USA 85:5879-83). An example of a linker suitable for use in an scFv is GGGGSGGGGSGGGGS (SEQ ID NO: 1). Other exemplary linkers contain at least 4-5 amino acids, ranging from about 4 residues to about 20 residues.

An exemplary format for the masked antigen binding protein comprises (i) two heavy chains comprising an scFv operably attached near the N-terminus of one or both heavy chain(s) and (ii) two light chains which associate with the heavy chains to form an antigen binding domain, wherein the scFv binds CD3 and the Fab portions bind EGFR. In this example, the antigen binding protein comprises three (or four) antigen binding domains, which typically bind two or more different antigens (or epitopes). An example of a linker suitable for linking an scFv to a heavy chain variable region is GGGGS (SEQ ID NO: 2). Exemplary linkers contain at least 1 suitable linker having 4-5 amino acids.

The masked antigen binding protein comprises a masking domain (MD) that is coupled to the antibody (AB) (e.g., via covalent bond or another form of attachment). A masking domain (MD) comprises a masking peptide (or masking polypeptide) (MP) and a protein recognition site (PR). A masking peptide (or masking polypeptide) may be a stretch of amino acids that impedes binding of an antigen binding domain to its antigen. Generally, masking peptides, short sequences of 5-15 amino acids in length, are employed, although shorter and longer sequences (i.e., masking polypeptides) are also contemplated. Masking domains are further described in, e.g., International Publication No. Wo 2017/040344; U.S. Patent Publication No. 2015/0079088 (incorporated by reference in its entirety and in particular with respect to the disclosure of masking domains for use with antibodies that bind EGFR) and U.S. Patent Publication No. 2016/0194399 (incorporated by reference in its entirety and in particular with respect to the disclosure of masking domains for use with antibodies that bind CD3).

In some embodiments, the masking peptide (or masking polypeptide) (MP) is attached to the antigen binding domain (AB) via a protein recognition site (PR), which is optionally part of a larger linker sequence (i.e., a stretch of amino acids connecting the MP to the antigen binding protein). A PR functions as a substrate (or binding site) for a protein or a protease, preferably an extracellular protease. The PR may be selected based on a protein or a protease that is produced by a cell (including, e.g. tumor cells) that is in proximity to cells that express the target and/or produced by a cell (including, e.g. tumor cells) that is co-localized in tissue with the desired target of at least one AB of the masked antigen binding protein. In some embodiments, the protease is u-type plasminogen activator (uPA, also referred to as urokinase), legumain, and/or matriptase (also referred to as MT-SP1 or MTSP1). In some embodiments, the protease is a matrix metalloprotease (MMP). In some embodiments, the protease is one of the proteases described in Rawlings, N. and Salvesen, G. Handbook of Proteolytic Enzymes (Third Edition). Elsevier, 2013. ISBN: 978-0-12-382219-2. Alternatively, the MP is coupled to the antigen binding protein via a non-cleavable protein binding domain. In this regard, the PR is optionally an amino acid sequence that, upon interaction with, e.g., a protein or a protease, is conformationally changed such that the position of the MP is adjusted and the AB is free to bind the target.

In various aspects of the disclosure, a bispecific or multispecific masked antigen binding protein comprises a MD for each antigen binding portion of the construct (e.g., AB1, AB2, AB3, etc.). For example, a bispecific or multispecific masked antigen binding protein comprising two Fab portions and two scFvs can comprise two sets of MDs, which can independently be the same or different. To illustrate, a bispecific or multispecific masked antigen binding protein comprises (i) two scFvs that bind CD3 and a MD1 (optionally the same MD for each scFv) attached to each scFv, and (ii) two Fab portions that bind EGFR and a MD2 attached to each Fab (optionally the same MD for each Fab). In various aspects, the PRs of MD1 and MD2 comprise the same protein recognition sequence, allowing release of the MD for each binding region in the same environment after engagement with a protease. The MD may be attached at the N-terminus or C-terminus of an antigen binding domain, so long as the MD is able to impede binding of the antigen binding domain to the target and the linker does not interfere with binding once the MD is released. When the antigen binding domain is a Fab, the MD may be operably linked to the heavy chain variable region or the light chain variable region. In instances where the bispecific or multispecific masked antigen binding protein comprises an intact antibody having both a Fab antigen binding domain and an scFv fused to the heavy chain (i.e., a "stacked" conformation), the MD associated with the Fab antigen binding domain is preferably fused to the light chain variable region.

In some embodiments, one AB domain (e.g., AB1) in the bispecific or multispecific masked antigen binding protein engages a T-cell surface ligand, such as CD3, and optionally takes the form of an scFv. Exemplary masked antigen binding proteins that engage CD3 include, but are not limited to, International Publication No. WO 2017/040344, U.S. Patent Publication No. 20160194399 (incorporated by reference in its entirety).

In some embodiments, one AB domain (e.g., AB2) in the antigen binding protein engages a tumor antigen, such as EGFR. Exemplary masked antigen binding proteins that engage EGFR include, but are not limited to, those antibodies disclosed in U.S. Patent Publication No. 20150079088, the disclosure of which is incorporated herein by reference in its entirety.

In some embodiments, the masked antigen binding protein (including, e.g., a bispecific or a multispecific masked antigen binding protein) is modified to alter the isoelectric point of the antibody. For example, in some embodiments, one or more negatively charged, pH sensitive amino acids (e.g., aspartic acid or glutamic acid) in one or both masking domains is substituted with a positively charged amino acid (e.g., a lysine or arginine). In some embodiments, replacing an aspartic acid in one or more masking moieties of the antibody may increase the pI. In some embodiments, the one or more negatively charged, pH sensitive amino acids (e.g., aspartic acid or glutamic acid) in one or both masking domains is removed or substituted with a neutral amino acid.

While the masked antigen binding protein of the disclosure (including, e.g., a bispecific or a multispecific masked antigen binding protein) is often described herein as comprising an intact antibody structure, the disclosure also contemplates the use of antigen binding antibody fragments that lack at least a portion of the traditional two heavy chain/two light chain structure. The fragment employed as a masked antigen binding protein comprises an antigen binding domain (AB) that is operably linked to a masking domain (MD), as described above. For example, in some embodiments, the antigen binding protein comprises two scFvs connected by a suitable linker (e.g., stretch of amino acids of sufficient length to allow each scFv to bind its target). One or both scFvs are attached to a MD; when both scFvs are attached to MDs, the MDs may be the same or different (i.e., the MP and/or PR are different although, in various aspects, it is preferable for the PR to be the same).

In some embodiments, the pharmaceutical composition or formulation comprises a masked antigen binding protein (e.g., a bispecific or multispecific masked antigen binding protein) in an amount ranging from about 50 µg to about 200 mg (or from about 500 µg to about 150 mg, or from about 50 mg to about 200 mg, or about 50 mg to about 150 mg, or about 50 mg to about 100 mg, or about 50 mg to about 75 mg). In some embodiments, the pharmaceutical composition comprises a masked antigen binding protein (including a bispecific or multispecific masked antibody-binding protein) in an amount of about 50 µg, about 100 µg, about 150 µg, about 200 µg, about 250 µg, about 300 µg, about 350 µg, about 400 µg, about 450 µg, about 500 µg, about 550 µg, about 600 µg, about 650 µg, about 700 µg, about 750 µg, about 800 µg, about 850 µg, about 900 µg, about 950 µg, about 1 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95, mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg or about 200 mg.

In some embodiments, the pharmaceutical composition comprises a masked antigen binding protein (e.g., a bispecific or multispecific masked antigen binding protein) in a concentration ranging from about 0.1 to about 50 mg/mL (or from about 1 to about 10 mg/mL or from about 10 to about 20 mg/mL, or from 20 mg/mL to about 50 mg.mL, or from about 1 to about 17 mg/mL, or from about 0.5 to about 5 mg/mL or from about 1 to about 5 mg/mL, or from about 3 to about 6 mg/mL). In some embodiments, the pharmaceutical composition comprises a masked antigen binding protein in a concentration of about 0.1 mg/mL, about 0.5 mg/mL, about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, about 20 mg/mL, about 21 mg/mL, about 22 mg/mL, about 23 mg/mL, about 24 mg/mL, about 25 mg/mL, about 26 mg/mL, about 27 mg/mL, about 28 mg/mL, about 29 mg/mL, about 30 mg/mL, about 31 mg/mL, about 32 mg/mL, about 33 mg/mL, about 34 mg/mL, about 35 mg/mL, about 36 mg/mL, about 37 mg/mL, about 38 mg/mL, about 39 mg/mL, about 40 mg/mL, about 41 mg/mL, about 42 mg/mL, about 43 mg/mL, about 44 mg/mL, about 45 mg/mL, about 46 mg/mL, about 47 mg/mL, about 48 mg/mL, about 49 mg/mL or about 50 mg/mL.

### Dosages and Therapeutic Use of the Formulation

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts or doses effective for this use will depend on the condition to be treated (the indication), the delivered antibody construct, the therapeutic context and objectives, the severity of the disease, prior therapy, the patient's clinical history and response to the therapeutic agent, the route of administration, the size (body weight, body surface or organ size) and/or condition (the age and general health) of the patient, and the general state of the patient's own immune system. The proper dose can be adjusted according to the judgment of the attending physician such that it can be administered to the patient once or over a series of administrations, and in order to obtain the optimal therapeutic effect.

A therapeutic effective amount of a masked antigen binding protein (e.g., a bispecific or multispecific masked antigen binding protein) preferably results in a decrease in severity of disease symptoms, an increase in frequency or duration of disease symptom-free periods, or a prevention of impairment or disability due to the disease affliction. For treating cancer, tumors, or malignant cells that express an antigen that is recognized by a masked antigen binding protein (including a bispecific or multispecific masked antigen binding protein) described herein,, a therapeutically effective amount of the masked antigen binding protein, e.g., an anti-tumor cell antigen (e.g., EGFR)/anti-CD3 bispecific or multispecific masked antigen binding protein, preferably inhibits cell division or tumor growth by at least about 20%, at least about 40%, at least about 50%, at least about 60%, at least about 70%, at least about 80%, or at least about 90% relative to untreated patients. The ability of a molecule to inhibit tumor growth may be evaluated in, e.g., an animal model predictive of efficacy.

The pharmaceutical compositions and formulations described herein are useful in the treatment, amelioration and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease. The term "disease" refers to any condition that would benefit from treatment with the pharmaceutical composition described herein. This includes chronic and acute disorders or diseases including those pathological conditions that predispose the mammal to the disease in question. The term "amelioration" as used herein refers to any improvement of the disease state (e.g., of a patient having a tumor or cancer or a metastatic cancer) by the administration of a masked antigen binding protein to a subject in need thereof. In the context of cancer, such an improvement may be seen as, for example, a slowing or stopping of the progression of the tumor or cancer or metastatic cancer of the patient. The term "prevention" as used herein means the avoidance of the occurrence or re-occurrence of disease or disorder or symptom thereof. For example, in the context of cancer, "prevention" encompasses the avoidance of occurrence or re-occurrence of a tumor or cancer or a metastatic cancer in the subject.

In some embodiments, the disclosure provides a method for the treatment of a disorder or disease associated with antigen (or antigens) recognized by the masked antigen binding protein described herein.

In one embodiment the disclosure provides a method for the treatment of a proliferative disease, e.g., cancer or a tumorous disease, comprising the step of administering to a subject in need thereof the pharmaceutical composition described herein. Malignant neoplasms (commonly called cancer) usually invade and destroy the surrounding tissue and may form metastases, i.e., they spread to other parts, tissues or organs of the body. Hence, the term "metastatic cancer" encompasses metastases to other tissues or organs than the one of the original tumor. The terms "subject in need" or those "in need of treatment" includes those already with the disorder, as well as those in which the disorder is to be prevented. The subject in need or "patient" includes human and other mammalian subjects that receive either prophylactic or therapeutic treatment.

### Routes of Administration

Exemplary routes of administration include, but are not limited to, topical routes (such as epicutaneous, inhalational, nasal, opthalmic, auricular / aural, vaginal, mucosal); enteral routes (such as oral, gastrointestinal, sublingual, sublabial, buccal, rectal); and parenteral routes (such as intravenous, intraarterial, intraosseous, intramuscular, intracerebral, intracerebroventricular, epidural, intrathecal, subcutaneous, intraperitoneal, extra-amniotic, intraarticular, intracardiac, intradermal, intralesional, intrauterine, intravesical, intravitreal, transdermal, intranasal, transmucosal, intrasynovial, intraluminal). Preferably, the pharmaceutical composition is administered parenterally, e.g., intravenously, subcutaneously, or intramuscularly. Parenteral administration may be achieved by injection, such as bolus injection, or by infusion, such as continuous infusion. Administration may be achieved via depot for long-term release. In some embodiments, the pharmaceutical composition is administered intravenously by an initial bolus followed by a continuous infusion to maintain therapeutic circulating levels of drug product. In some embodiments, the pharmaceutical composition is administered as a one-time dose. Pharmaceutical compositions may be administered using a medical device. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

The disclosure contemplates a lyophilized composition have the features described herein. If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization. The disclosure contemplates a reconstituted composition of a previously lyophilized composition have the features described herein. The pharmaceutical composition can be administered as a sole therapeutic or in combination with additional therapies such as anti-cancer therapies as needed, e.g. other proteinaceous and non-proteinaceous drugs. These drugs may be administered simultaneously with the composition of the disclosure as defined herein or separately before or after administration of said formulation in timely defined intervals and doses.

### Kits

As an additional aspect, kits which comprise one or more pharmaceutical compositions described herein packaged in a manner which facilitates their use for administration to subjects are provided. In one embodiment, such a kit includes a pharmaceutical composition described herein (e.g., a composition comprising a masked antigen binding protein (e.g., a bispecific or multispecific masked antigen binding protein)) packaged in a container such as a sealed bottle, vessel, single-use or multi-use vial, prefilled syringe, or prefilled injection device, optionally with a label affixed to the container or included in the package that describes use of the compound or composition in practicing the method. In one aspect, the composition is packaged in a unit dosage form. The kit may further include a device suitable for administering the composition according to a specific route of administration. Preferably, the kit contains a label that describes use of a masked antigen binding protein described herein or pharmaceutical composition described herein.

Generally, various storage and/or dosage forms are conceivable for the pharmaceutical composition of the invention, depending, i.e., on the intended route of administration, delivery format and desired dosage (see, for example, Remington's Pharmaceutical Sciences, 22nd edition, Oslo, A., Ed., (2012)). The skilled person will be aware that such choice of a particular dosage form may for example influence the physical state, stability, rate of *in vivo* release and rate of in vivo clearance of an antibody.

### EXAMPLES

### EXAMPLE 1

### Impact of Saccharide Stabilizer (e.g., sucrose) and Low pH on Aggregation of Masked Antigen Binding Protein Compositions

A composition comprising a bispecific masked antigen binding protein (1 mg/mL) in 100 mM glycine, pH 4.9, was generated (Formulation A). This liquid composition showed significant aggregation immediately after storage at 2-8°C or four weeks at storage at 2-8°C with approximately 27-28% high molecular weight (HMW) species (see Figures 6, 7 and 10).

To improve the clinical utility of the composition by improving stability and reducing aggregation and the amount of HMW species, the composition was buffer-exchanged into several formulations (Formulations B-F) ranging from pH 3.6 to pH 7.0 as follows:
Formulation B: 10 mM glutamate, 9% sucrose, pH 3.6
Formulation C: 10 mM glutamate, 9% sucrose, pH 4.2;
Formulation D: 10 mM acetate, 9% sucrose, pH 5.2;
Formulation E: 10 mM phosphate, 9% sucrose, pH 6.3; and
Formulation F: 10 mM phosphate, 9% sucrose, pH 7.0.

The buffer exchange was performed by dialyzing the starting composition in a 7000 kDa cutoff cassette at 4°C overnight with three buffer changes. The dialyzed material was vialed after the addition of 0.01% polysorbate 80, and the stability of the samples was monitored for one month at 2-8°C and 25°C. The final concentration of the vialed masked antigen binding protein in each formulation was 1 mg/mL. All samples were analyzed by size exclusion ultra high performance liquid chromatography (SE-UHPLC), at two weeks and four weeks at 2-8°C and 25°C, respectively. The results of the SE-UHPLC analysis of Formulations B-F are set forth in Figures 1-6, respectively. The results of the SE-UHPLC analysis of Formulation A is set forth in Figure 10.

As shown in Figure 6A, the % high molecular weight (HMW) species significantly decreased in the lower pH formulations after four weeks of storage at 2-8°C compared to starting composition (i.e., Formulation A). For example, the masked antigen binding protein formulations described herein ranked from highest to lowest amount of observed aggregation is Formulation A > Formulation F > Formulation E > Formulation D > Formulation C > Formulation B.

This data strongly suggests that the stability, aggregation and clinical utility of the pharmaceutical composition containing a masked antigen binding protein is significantly improved by: (1) the addition of a saccharide or stabilizer (e.g., sucrose) - where the inclusion a saccharide or stabilizer (e.g., sucrose) reduced aggregation / % HMW species by ~3-fold; and (2) formulating the pharmaceutical composition in a low pH, where the pH is below the isoelectric point (pI) of the AB1, AB2, MD1 and MD2 regions of the masked antigen binding protein - which further reduced aggregation / % HMW species by another ~3-fold. The pIs of the AB1, AB2, MD1 and MD2 regions of the bispecific masked antigen binding protein were determined using the Bjellqvist algorithm to have a pattern comparable to the bispecific masked antigen binding protein 2 provided in Table 1. When formulated at pH greater than 4.54 it appears that a negatively charged MD1 caps an otherwise positively charged protein potentially resulting in solution instability. At the lower pH of 4, all the components (e.g., AB1, AB2, MD1 and MD2) are positively charged to varying degrees thereby creating repulsive forces. This data strongly supports that the combination of a saccharide or stabilizer (e.g., sucrose) and low pH (where the pH is below the isoelectric point (pI) of the AB 1, AB2, MD1 and/or MD2 regions of the masked antigen binding protein) synergistically or dramatically reduces the aggregation / % HMW species of pharmaceutical compositions comprising a masked antigen binding protein (including, e.g., bispecific or multispecific masked antigen binding proteins).

### EXAMPLE 2

### Aggregation of Bispecific Masked antigen binding protein Compositions is Reversed by Addition of a Saccharide or Stabilizer (e.g., sucrose) and low pH

A sample of the bispecific masked antigen binding protein with high starting levels of aggregation was buffer exchanged into the low pH formulation buffer (Formulation C), as described in Example 1. Briefly, the buffer exchange was performed by dialyzing the starting material in a 7000 kDa cutoff cassette at 4°C overnight with three buffer changes. The dialysis process resulted in a significant decrease in aggregation levels, indicating that the low pH formulation is capable of reversing the initial aggregation. Compare time zero of Figure 2B reporting 3.91% HMW species in Formulation C vs. Figure 10B reporting 28.49% HMW species for Formulation A. This comparison is also shown in Figure 7B. The samples were analyzed by size exclusion ultra high performance liquid chromatography (SE-UHPLC), a technique that separates proteins in solution based on their hydrodynamic volume using a size exclusion ultra high performance analytical column.

As shown in Figures 8A-8B, the dialyzed material at 1 mg/mL was stable after 0, 1 weeks and two weeks of storage at 4°C and 25°C. There was no increase in aggregation levels post-storage. The samples stored at 25°C exhibited a slight decrease in high molecular species by SEC, suggesting that the aggregation could be driven by hydrophobic interactions.

The low pH formulation (Formulation C) was also effective at stabilizing the bispecific masked antigen binding protein at higher concentrations. The protein in the low pH formulation buffer (Formulation C) at 17 mg/mL had significantly lower aggregation compared to the starting material (Formulation A) at 11 mg/mL (Figure 9). The 17 mg/mL material was stable after 0, two weeks and 4 weeks of storage at 4°C (Figure 9). Even though Formulation C displayed significant reduction in aggregation / % HMW species and improved stability at 2-8°C or 25°C at 0, 2 weeks and 4 weeks (Figure 2) compared to Formulation A (Figure 10), Formulation B at pH 3.6 showed additional reduction in aggregation / % HMW species compared to Formulation C. See, e.g., Figure 6; see also Figure 1 and compare to Figure 2). This data shows that further reducing the pH of the pharmaceutical composition - which is already below the isoelectric point (pI) of the AB1, AB2, MD1 and MD2 regions of the masked antigen binding protein - further reduces aggregation of the masked antigen binding protein.

### EXAMPLE 3

### Impact of Polyol (e.g., sucrose) and Low pH on Aggregation of Masked Antigen Binding Protein Compositions

The pI of the antibody domain (AB 1 and/or AB2) and the bulking agent/masking domain (MD 1 and/or MD2) of a ProTIA prodrug comprising amino acid sequences as described in Tables 5, 10 and/or 12 and/or Figures 36 or 37 of International Publication No. WO 2017/040344 is determined. Next, the ProTIA prodrug is formulated in a suitable buffer (such as 10mM glutamate or 10mM acetate), along with a suitable polyol (such as 9% sucrose), optionally with a surfactant (such as 0.01% polysorbate 80) and the pH of the composition is lowered to between about 7.0-3.5, such that the pH of the composition is below the pI of the antibody domain (AB 1 and/or AB2) and the bulking agent/masking domain (MD1 and/or MD2). The impact of the polyol and low pH on the aggregation of the ProTIA prodrug low pH will be assessed as described in Examples 1 and 2.

### ASPECTS OF DISCLOSURE:

1. A pharmaceutical composition comprising:
   (a) a masked antigen binding protein comprising:
      (i) an antibody or antigen binding fragment thereof (AB1) that binds to an antigen; and
      (ii) a masking domain (MD1) coupled to AB1, wherein the masking domain comprises:
         a. a masking peptide or masking polypeptide (MP1) that inhibits or reduces the binding of AB1 to the antigen; and
         b. a protein recognition site (PR1), wherein binding to or cleavage of the PR1 by a protein or a protease increases AB1 binding to the antigen;
   (b) at least one buffer agent; and
   (c) at least one polyol,
   wherein the pH of the pharmaceutical composition is below the isoelectric point (pI) of the AB1 and MD1 regions of the masked antigen binding protein.
2. The pharmaceutical composition of aspect 1, wherein the masked antigen binding protein further comprises:
   (iii) a second antibody or antigen binding fragment thereof (AB2) that binds to a second antigen; and
   (iv) a second masking domain (MD2) coupled to AB2, wherein the second masking domain comprises:
      a. a masking peptide or masking polypeptide (MP2) that inhibits or reduces the binding of AB2 to the antigen; and
      b. a protein recognition site (PR2), wherein binding to or cleavage of the PR2 by a protein or a protease increases AB2 binding to the second antigen,
   wherein the pH of the pharmaceutical composition is below the isoelectric points (pI) of the AB1, AB2, MD1 and MD2 regions of the masked antigen binding protein.
3. The pharmaceutical composition of aspects 1 or 2, wherein the masked antigen binding protein is a Probody or a ProTIA prodrug.
4. The pharmaceutical composition of any one aspects 1-3, wherein AB1 and/or AB2 is an scFv or a Fab.
5. The pharmaceutical composition of any one aspects 1-4, wherein AB1 and/or AB2 binds an antigen expressed on the surface of a cell.
6. The pharmaceutical composition of any one of aspects 1-5, wherein the at least one buffer agent is selected from the group consisting of acetate, glutamate, citrate, succinate, tartrate, fumarate, maleate, histidine, phosphate, and 2-(N-morpholino) ethanesulfonate or a combination thereof.
7. The pharmaceutical composition of aspect 6, wherein the at least one buffer agent is present at a concentration range of about 5 to about 200 mM.
8. The pharmaceutical composition of aspect 7, wherein the at least one buffer agent is present at a concentration range of about 10 to about 50 mM.
9. The pharmaceutical composition of any one of aspects 1-8, wherein the at least one polyol is selected from the group consisting of a saccharide, a cyclic polysaccharide, a sugar alcohol, a linear branched dextran, and a linear non-branched dextran, or combinations thereof.
10. The pharmaceutical composition of aspect 9, wherein the saccharide is a monosaccharide or a disaccharide.
11. The pharmaceutical composition of any one aspects 1-10, wherein the polyol is selected from the group consisting of sucrose, trehalose, mannitol, sorbitol, xylitol, erythitol, isomalt, glycerol, a cyclodextrin, captisol or a combination thereof.
12. The pharmaceutical composition of any one of aspects 1-11, wherein the at least one polyol is present at a concentration in the range of about 1 to about 20% (w/V).
13. The pharmaceutical composition of aspect 12, wherein the at least one polyol is present at a concentration in the range of about 9 to about 12% (w/V).
14. The pharmaceutical composition of any one of aspects 1-13, further comprising at least one surfactant.
15. The pharmaceutical composition of aspect 14, wherein the at least one surfactant is selected from the group consisting of polysorbate 20, polysorbate 40, polysorbate 60, polysorbate 80, poloxamer 188, pluronic F68, triton X-100, polyoxyethylen3, and PEG 3350, PEG 4000, and PEG 8000 or a combination thereof.
16. The pharmaceutical composition of aspect 14 or 15, wherein the at least one surfactant is present at a concentration in the range of about 0.001 to about 0.5 % (w/V).
17. The pharmaceutical composition of aspect 16, wherein the at least one surfactant is present at a concentration in the range of about 0.001 to about 0.01% (w/V).
18. The pharmaceutical composition of any one of aspects 1-17, wherein the pH of the composition is about 2 pH units lower than the pI of AB1, AB2, MD1, and/or MD2.
19. The pharmaceutical composition of any one of aspects 1-17, wherein the pH of the composition is greater than about 3.5.
20. The pharmaceutical composition of any one of aspects 1-19, wherein the pH of the composition is between 7.0 and 3.6.
21. The pharmaceutical composition of any one of aspects 1-20, wherein the pH of the composition is between 4.0 and 5.0.
22. The pharmaceutical composition of any one of aspects 1-21, wherein the pH of the composition is between 3.5 and 5.5.
23. The pharmaceutical composition of any one of aspects 1-22, wherein the pH of the composition is about 4.2 or 3.6.
24. The pharmaceutical composition of any one of aspects 1-23, having an osmolarity in the range of 150 to 500 mOsm.
25. The pharmaceutical composition of any one of aspects 1-24, further comprising an amino acid excipient.
26. The pharmaceutical composition of aspect 25, wherein the amino acid excipient is present in the concentration range of about 0.1 to about 20 mM.
27. The pharmaceutical composition of any one of aspects 1-26, wherein the composition comprises 10 mM glutamate, 9% (w/V) sucrose and 0.01% (w/V) polysorbate 80, and wherein the pH of the pharmaceutical composition is 4.2 or 3.6.
28. The pharmaceutical composition of any one of aspects 1-27, wherein the masked antigen binding protein is present in a concentration range of about 0.1 to about 30 mg/ml.
29. The pharmaceutical composition of any one of aspects 1-28, wherein the masked antigen binding protein is present in an amount ranging from about 50 µg to about 200 mg.
30. The pharmaceutical composition of any one of aspects 1-29, wherein the pharmaceutical composition is a lyophilized composition.
31. The pharmaceutical composition of any one of aspects 1-30, wherein the pharmaceutical composition is a liquid composition.
32. The pharmaceutical composition of aspect 31, wherein the pharmaceutical composition is a reconstituted lyophilized composition.
33. The pharmaceutical composition of any one of aspects 1-32, wherein the composition is a liquid composition and is stable after storage at 2-8°C, 4°C and/or 25°C for 0 weeks, 1 week, 2, weeks, 4 weeks, 2 months, 6 months, 1 year and/or 2 years; or wherein the composition is a lyophilized composition and is stable after storage at 2-8°C, 4°C and/or 25°C for 0 weeks, 1 week, 2, weeks, 4 weeks, 2 months, 6 months, 1 year, 2 years, 3 years, 4 years and/or 5 years.
34. The pharmaceutical composition of any one of aspects 1-33, wherein the amount of antibody aggregation is reduced.
35. The pharmaceutical composition of any one of aspects 1-34, wherein the amount of high-molecular weight antibody species is reduced as measured by ultra-high performance liquid chromatography (SE-UHPLC).
36. The pharmaceutical composition of any one of aspects 1-35, wherein the amount of antibody aggregation and/or high-molecular weight antibody species is reduced by about 1-fold, about 2-fold, about 3-fold, about 4-fold, about 5-fold, about 6-fold, about 7-fold, about 8-fold, about 9-fold or about 10-fold compared to a reference pharmaceutical composition at a higher pH than the pharmaceutical composition.
37. The pharmaceutical composition of any one of aspects 1-36, wherein the pI is measured or determined using the Bjellqvist algorithm.
38. A method of treating cancer in a subject in need thereof comprising administering the composition of any one of aspects 1-37 to the subject.

## Claims

1. A pharmaceutical composition comprising:
(a) a masked antigen binding protein comprising:
(i) an antibody or antigen binding fragment thereof (AB1) that binds to an antigen; and
(ii) a masking domain (MD1) coupled to AB1, wherein the masking domain comprises:
a. a masking peptide or masking polypeptide (MP1) that inhibits or reduces the binding of AB1 to the antigen; and
b. a protein recognition site (PR1), wherein binding to or cleavage of the PR1 by a protein or a protease increases AB1 binding to the antigen;
(b) at least one buffer agent; and
(c) at least one polyol,
wherein the pH of the pharmaceutical composition is between 3.5 and 7.0.

2. The pharmaceutical composition of claim 1, wherein the masked antigen binding protein further comprises:
(iii) a second antibody or antigen binding fragment thereof (AB2) that binds to a second antigen; and
(iv) a second masking domain (MD2) coupled to AB2, wherein the second masking domain comprises:
a. a masking peptide or masking polypeptide (MP2) that inhibits or reduces the binding of AB2 to the antigen; and
b. a protein recognition site (PR2), wherein binding to or cleavage of the PR2 by a protein or a protease increases AB2 binding to the second antigen.

3. The pharmaceutical composition of claims 1 or 2, wherein the masked antigen binding protein is a Probody or a ProTIA prodrug.

4. The pharmaceutical composition of any one of claims 1-3, wherein AB1 and/or AB2 is:
(i) an scFv or a Fab; and/or
(ii) binds an antigen expressed on the surface of a cell.

5. The pharmaceutical composition of any one of claims 1-4, wherein the at least one buffer agent is:
(i) selected from the group consisting of acetate, glutamate, citrate, succinate, tartrate, fumarate, maleate, histidine, phosphate, and 2-(N-morpholino) ethanesulfonate or a combination thereof; and/or
(ii) present at a concentration range of about 5 to about 200 mM or about 10 to about 50 mM.

6. The pharmaceutical composition of any one of claims 1-5, wherein the at least one polyol is selected from the group consisting of a saccharide, a cyclic polysaccharide, a sugar alcohol, a linear branched dextran, and a linear non-branched dextran, or combinations thereof, optionally wherein the saccharide is a monosaccharide or a disaccharide.

7. The pharmaceutical composition of any one of claims 1-6, wherein the polyol is selected from the group consisting of sucrose, trehalose, mannitol, sorbitol, xylitol, erythitol, isomalt, glycerol, a cyclodextrin, captisol or a combination thereof; and/or wherein the at least one polyol is present at a concentration in the range of about 1 to about 20% (w/V), optionally, about 9 to about 12% (w/V).

8. The pharmaceutical composition of any one of claims 1-7, further comprising at least one surfactant, optionally wherein:
(i) the at least one surfactant is selected from the group consisting of polysorbate 20, polysorbate 40, polysorbate 60, polysorbate 80, poloxamer 188, pluronic F68, triton X-100, polyoxyethylen3, and PEG 3350, PEG 4000, and PEG 8000 or a combination thereof; and/or
(ii) the at least one surfactant is present at a concentration in the range of about 0.001 to about 0.5 % (w/V), or about 0.001 to about 0.01% (w/V).

9. The pharmaceutical composition of any one of claims 1-8, wherein the pH of the composition is:
(i) between 7.0 and 3.6,
(ii) between 3.5 and 5.5,
(iii) from 3.5 to 5.0,
(iv) from 4.0 to 5.5,
(v) between 4.0 and 5.0,
(vi) from 4.2 to 4.8,
(vii) from 3.6 to 4.2,
or
(viii) about 4.2 or 3.6.

10. The pharmaceutical composition of any one of claims 1-9, having an osmolarity in the range of 150 to 500 mOsm.

11. The pharmaceutical composition of any one of claims 1-10, further comprising an amino acid excipient, optionally wherein the amino acid excipient is present in the concentration range of about 0.1 to about 20 mM.

12. The pharmaceutical composition of any one of claims 1-11, wherein the composition comprises 10 mM glutamate, 9% (w/V) sucrose and 0.01% (w/V) polysorbate 80, and wherein the pH of the pharmaceutical composition is 4.2 or 3.6.

13. The pharmaceutical composition of any one of claims 1-12, wherein:
(i) the masked antigen binding protein is present in a concentration range of about 0.1 to about 30 mg/ml, and/or
(ii) the masked antigen binding protein is present in an amount ranging from about 50 µg to about 200 mg, and/or
(iii) the pharmaceutical composition is a lyophilized composition, and/or
(iv) the pharmaceutical composition is a liquid composition, optionally, wherein the pharmaceutical composition is a reconstituted lyophilized composition, and/or
(v) the composition is a liquid composition and is stable after storage at 2-8°C, 4°C and/or 25°C for 0 weeks, 1 week, 2, weeks, 4 weeks, 2 months, 6 months, 1 year and/or 2 years; or wherein the composition is a lyophilized composition and is stable after storage at 2-8°C, 4°C and/or 25°C for 0 weeks, 1 week, 2, weeks, 4 weeks, 2 months, 6 months, 1 year, 2 years, 3 years, 4 years and/or 5 years, and/or
(vi) the amount of antibody aggregation is reduced, and/or
(vii) the amount of high-molecular weight antibody species is reduced as measured by ultra-high performance liquid chromatography (SE-UHPLC), and/or
(viii) the amount of antibody aggregation and/or high-molecular weight antibody species is reduced by about 1-fold, about 2-fold, about 3-fold, about 4-fold, about 5-fold, about 6-fold, about 7-fold, about 8-fold, about 9-fold or about 10-fold compared to a reference pharmaceutical composition at a higher pH than the pharmaceutical composition.

14. The composition of any one of claims 1-13 for use in treating cancer in a subject.

15. Use of
(a) at least one buffer agent that maintains a pH of between 3.5 and 7.0 and
(b) at least one polyol
for minimizing or reducing aggregation and/or high-molecular weight species of a masked antigen binding protein in a pharmaceutical composition;
wherein the masked antigen binding protein comprises
(i) an antibody or antigen binding fragment thereof (AB1) that binds to an antigen and
(ii) a masking domain (MD1) coupled to AB1, wherein the masking domain comprises a masking peptide or masking polypeptide (MP1) that inhibits or reduces the binding of AB1 to the antigen and a protein recognition site (PR1), wherein binding to or cleavage of the PR1 by a protein or a protease increases AB1 binding to the antigen.
